# EUROPEAN PATENT SPECIFICATION

(11) **EP 3 290 449 B1**
(45) Date of publication and mention of the grant of the patent: **05.06.2019**
(21) Application number: 16786152.5
(22) Date of filing: 28.04.2016
(51) Int. Cl.: C08F 136/08, C08F 4/54, C08F 2/44, C08F 4/60, C08F 36/08, C12P 5/02, B60C 1/00

(54) **METHOD FOR PRODUCING POLYISOPRENE**
VERFAHREN ZUR HERSTELLUNG VON POLYISOPREN
PROCÉDÉ DE PRODUCTION DE POLYISOPRÈNE

(30) Priority: 30.04.2015 JP 2015093585
(43) Date of publication of application: 07.03.2018
(73) Proprietor: Bridgestone Corporation, Tokyo 104-8340 (JP); Ajinomoto Co., Inc., Tokyo 104-8315 (JP)
(72) Inventor: NISHIURA, Fumiaki, Tokyo 104-8340 (JP); NISHIO, Yosuke, Kawasaki-shi Kanagawa 210-8681 (JP); RACHI, Hiroaki, Kawasaki-shi Kanagawa 210-8681 (JP); HIOKI, Satoshi, Kawasaki-shi Kanagawa 210-8681 (JP)
(74) Representative: Oxley, Robin John George
(86) International application number: PCT/JP2016/002240
(87) International publication number: WO 2016/174875

(56) References cited:
- WO-A1-2013/179722
- JP-A- 2009 215 429
- JP-A- 2011 526 943
- JP-A- 2013 216 850
- US-A- 3 329 666
- US-A1- 2003 212 220
- US-A1- 2004 009 870
- US-A1- 2005 137 338
- US-B2- 8 901 262

### SEQUENCE LISTING

<110> Bridgestone Corporation and Ajinomoto Company, Incorporated
<120> Isoprene containing composition
<130> 2015P01175WO
<150> JP 2015-093585
   <151> 2015-04-30
<160> 34
<210> 1
   <211> 2412
   <212> DNA
   <213> Artificial Sequence
   <223> EFMvaE
   <400> 1
   atgaaaaccg tggttattat cgatgcgctg cgcacgccga ttggtaaata taaaggcagc      60
   ctgtctcaag tgagcgccgt tgatctgggt acgcatgtga ccacgcagct gctgaaacgt     120
   cacagcacca tctctgaaga aattgatcag gtgatctttg gtaacgttct gcaagccggt     180
   aatggtcaga atccggcacg tcagattgca atcaacagtg gcctgagcca tgaaattccg     240
   gcgatgaccg tgaatgaagt ttgcggtagc ggcatgaaag cggttattct ggccaaacag     300
   ctgatccagc tgggtgaagc ggaagtgctg attgccggcg gtatcgaaaa catgagtcag     360
   gcaccgaaac tgcaacgttt taattatgaa accgaaagct acgatgcccc gttcagctct     420
   atgatgtatg atggcctgac cgatgcattt agcggtcagg cgatgggcct gacggcagaa     480
   aacgtggcgg aaaaatacca tgttacccgc gaagaacagg atcagttttc tgttcacagt     540
   cagctgaaag cggcccaggc ccaggcagaa ggtattttcg ccgatgaaat cgcaccgctg     600
   gaagtgtctg gtacgctggt tgaaaaagat gaaggcattc gtccgaatag tagcgtggaa     660
   aaactgggca ccctgaaaac ggtgttcaaa gaagatggca ccgttacggc gggcaatgca     720
   agcaccatca atgatggtgc gagtgccctg attatcgcga gccaggaata tgcagaagcg     780
   catggcctgc cgtacctggc cattatccgc gattctgtgg aagttggtat tgatccggca     840
   tatatgggca ttagtccgat caaagcgatt cagaaactgc tggcccgtaa ccagctgacc     900
   accgaagaaa ttgatctgta cgaaatcaat gaagcgtttg cagcgaccag tattgtggtt     960
   cagcgcgaac tggccctgcc ggaagaaaaa gttaacattt atggcggtgg catcagcctg    1020
   ggtcacgcaa ttggtgccac cggtgcacgt ctgctgacca gtctgagcta tcagctgaat    1080
   cagaaagaga aaaaatacgg tgtggcaagc ctgtgtattg gtggcggtct gggtctggcc    1140
   atgctgctgg aacgtccgca gcagaagaaa aactctcgtt tttaccagat gagtccggaa    1200
   gaacgtctgg ccagtctgct gaacgaaggc cagattagcg cagataccaa aaaagaattc    1260
   gaaaatacgg cactgtctag tcagatcgcg aaccatatga ttgaaaatca gatcagcgaa    1320
   accgaagtgc cgatgggtgt tggcctgcac ctgaccgtgg atgaaacgga ttatctggtt    1380
   ccgatggcga cggaagaacc gagcgttatt gccgcactgt ctaacggtgc aaaaatcgcg    1440
   cagggcttta aaaccgtgaa tcagcagcgt ctgatgcgcg gccagattgt gttctacgat    1500
   gttgcggatc cggaaagcct gatcgataaa ctgcaagtgc gcgaagccga agtttttcag    1560
   caggcagaac tgagctatcc gtctattgtg aaacgtggcg gtggcctgcg cgatctgcaa    1620
   taccgtacct ttgatgaaag tttcgtgagc gttgatttcc tggtggatgt taaagatgcc    1680
   atgggtgcaa acatcgtgaa tgcgatgctg gaaggcgttg ccgaactgtt tcgtgaatgg    1740
   ttcgcggaac agaaaatcct gttttctatc ctgagtaact acgcgaccga aagcgtggtt    1800
   accatgaaaa cggccattcc tgtgagccgc ctgtctaaag gtagtaatgg ccgtgaaatt    1860
   gcggaaaaaa tcgttctggc gagccgctat gcctctctgg atccgtaccg tgccgtgacc    1920
   cataacaaag gtattatgaa tggcatcgaa gcagtggttc tggcgaccgg taacgatacc    1980
   cgtgccgtgt ctgcaagttg ccatgcattc gcagttaaag aaggtcgtta tcagggcctg    2040
   accagctgga cgctggatgg tgaacagctg atcggcgaaa tttctgtgcc gctggccctg    2100
   gcaaccgtgg gtggcgcgac gaaagttctg ccgaaaagcc aggcggccgc agatctgctg    2160
   gcggtgaccg atgcaaaaga actgtctcgc gtggttgcgg ccgttggtct ggcacagaat    2220
   ctggcagcgc tgcgtgcgct ggtgtctgaa ggtattcaga aaggccacat ggcactgcaa    2280
   gcccgtagtc tggccatgac cgtgggtgca acgggcaaag aagtggaagc agttgcgcag    2340
   cagctgaaac gccagaaaac catgaaccag gatcgtgcca tggcaatcct gaatgatctg    2400
   cgcaaacagt aa                                                        2412
<210> 2
   <211> 1152
   <212> DNA
   <213> Artificial Sequence
<223> EFMvaS
<400> 2
   atgaccattg gtatcgataa aattagcttt ttcgtgccgc cgtattacat cgatatgacg 60
   gcgctggccg aagcacgtaa cgttgatccg ggcaaatttc atattggcat cggtcaggat 120
   cagatggcgg tgaacccgat ttctcaggat atcgttacct tcgcggccaa tgcagcggaa 180
   gcaattctga cgaaagaaga taaagaagcg attgatatgg tgatcgttgg caccgaaagc 240
   tctatcgatg aaagtaaagc cgcagcggtg gttctgcacc gtctgatggg cattcagccg 300
   tttgcgcgca gcttcgaaat caaagaagcc tgctatggcg cgaccgccgg tctgcaactg 360
   gccaaaaacc atgtggcact gcacccggat aaaaaagttc tggtggttgc cgcagatatt 420
   gcgaaatacg gtctgaatag cggcggtgaa ccgacccagg gtgcaggtgc cgtggcaatg 480
   ctggttgcat ctgaaccgcg tattctggcg ctgaaagaag ataacgtgat gctgacccag 540
   gatatctatg atttttggcg tccgaccggt catccgtacc cgatggtgga tggcccgctg 600
   agtaatgaaa cctatattca gagcttcgcc caggtttggg atgaacataa aaaacgtacg 660
   ggtctggatt ttgcggatta tgatgcactg gcgttccaca ttccgtacac caaaatgggc 720
   aaaaaagcgc tgctggccaa aatcagcgat cagacggaag ccgaacagga acgtattctg 780
   gcacgctatg aagaaagcat cgtgtactct cgtcgcgttg gcaacctgta taccggttct 840
   ctgtacctgg gcctgattag tctgctggaa aacgcgacca cgctgacggc cggcaatcag 900
   atcggtctgt tttcttatgg cagtggtgcc gtggcagaat ttttcaccgg tgaactggtt 960
   gccggctacc agaaccatct gcaaaaagaa acccacctgg ccctgctgga taatcgcacg 1020
   gaactgtcta ttgcagaata tgaagcaatg tttgcggaaa ccctggatac ggatatcgat 1080
   cagaccctgg aagatgaact gaaatatagt attagcgcga tcaacaatac ggtgcgtagt 1140
   taccgcaatt aa 1152
<210> 3
   <211> 5314
   <212> DNA
   <213> Artificial Sequence
   <223> pUC57-EFmvaE
   <400> 3
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt cgagctcggt accttgacaa 420
   ttaatcatcc ggctcgtata atgtgtggaa ttgtgagcgg ataacaattt cacacaggaa 480
   acagcgccgc tgagaaaaag cgaagcggca ctgctcttta acaatttatc agacaatctg 540
   tgtgggcact cgaccggaat tatcgattaa ctttattatt aaaaattaaa gaggtatata 600
   ttaatgtatc gattaaataa ggaggaataa accatggatc cgagctcagg aggtaaaaaa 660
   acatgaaaac cgtggttatt atcgatgcgc tgcgcacgcc gattggtaaa tataaaggca 720
   gcctgtctca agtgagcgcc gttgatctgg gtacgcatgt gaccacgcag ctgctgaaac 780
   gtcacagcac catctctgaa gaaattgatc aggtgatctt tggtaacgtt ctgcaagccg 840
   gtaatggtca gaatccggca cgtcagattg caatcaacag tggcctgagc catgaaattc 900
   cggcgatgac cgtgaatgaa gtttgcggta gcggcatgaa agcggttatt ctggccaaac 960
   agctgatcca gctgggtgaa gcggaagtgc tgattgccgg cggtatcgaa aacatgagtc 1020
   aggcaccgaa actgcaacgt tttaattatg aaaccgaaag ctacgatgcc ccgttcagct 1080
   ctatgatgta tgatggcctg accgatgcat ttagcggtca ggcgatgggc ctgacggcag 1140
   aaaacgtggc ggaaaaatac catgttaccc gcgaagaaca ggatcagttt tctgttcaca 1200
   gtcagctgaa agcggcccag gcccaggcag aaggtatttt cgccgatgaa atcgcaccgc 1260
   tggaagtgtc tggtacgctg gttgaaaaag atgaaggcat tcgtccgaat agtagcgtgg 1320
   aaaaactggg caccctgaaa acggtgttca aagaagatgg caccgttacg gcgggcaatg 1380
   caagcaccat caatgatggt gcgagtgccc tgattatcgc gagccaggaa tatgcagaag 1440
   cgcatggcct gccgtacctg gccattatcc gcgattctgt ggaagttggt attgatccgg 1500
   catatatggg cattagtccg atcaaagcga ttcagaaact gctggcccgt aaccagctga 1560
   ccaccgaaga aattgatctg tacgaaatca atgaagcgtt tgcagcgacc agtattgtgg 1620
   ttcagcgcga actggccctg ccggaagaaa aagttaacat ttatggcggt ggcatcagcc 1680
   tgggtcacgc aattggtgcc accggtgcac gtctgctgac cagtctgagc tatcagctga 1740
   atcagaaaga gaaaaaatac ggtgtggcaa gcctgtgtat tggtggcggt ctgggtctgg 1800
   ccatgctgct ggaacgtccg cagcagaaga aaaactctcg tttttaccag atgagtccgg 1860
   aagaacgtct ggccagtctg ctgaacgaag gccagattag cgcagatacc aaaaaagaat 1920
   tcgaaaatac ggcactgtct agtcagatcg cgaaccatat gattgaaaat cagatcagcg    1980
   aaaccgaagt gccgatgggt gttggcctgc acctgaccgt ggatgaaacg gattatctgg    2040
   ttccgatggc gacggaagaa ccgagcgtta ttgccgcact gtctaacggt gcaaaaatcg    2100
   cgcagggctt taaaaccgtg aatcagcagc gtctgatgcg cggccagatt gtgttctacg    2160
   atgttgcgga tccggaaagc ctgatcgata aactgcaagt gcgcgaagcc gaagtttttc    2220
   agcaggcaga actgagctat ccgtctattg tgaaacgtgg cggtggcctg cgcgatctgc    2280
   aataccgtac ctttgatgaa agtttcgtga gcgttgattt cctggtggat gttaaagatg    2340
   ccatgggtgc aaacatcgtg aatgcgatgc tggaaggcgt tgccgaactg tttcgtgaat    2400
   ggttcgcgga acagaaaatc ctgttttcta tcctgagtaa ctacgcgacc gaaagcgtgg    2460
   ttaccatgaa aacggccatt cctgtgagcc gcctgtctaa aggtagtaat ggccgtgaaa    2520
   ttgcggaaaa aatcgttctg gcgagccgct atgcctctct ggatccgtac cgtgccgtga    2580
   cccataacaa aggtattatg aatggcatcg aagcagtggt tctggcgacc ggtaacgata    2640
   cccgtgccgt gtctgcaagt tgccatgcat tcgcagttaa agaaggtcgt tatcagggcc    2700
   tgaccagctg gacgctggat ggtgaacagc tgatcggcga aatttctgtg ccgctggccc    2760
   tggcaaccgt gggtggcgcg acgaaagttc tgccgaaaag ccaggcggcc gcagatctgc    2820
   tggcggtgac cgatgcaaaa gaactgtctc gcgtggttgc ggccgttggt ctggcacaga    2880
   atctggcagc gctgcgtgcg ctggtgtctg aaggtattca gaaaggccac atggcactgc    2940
   aagcccgtag tctggccatg accgtgggtg caacgggcaa agaagtggaa gcagttgcgc    3000
   agcagctgaa acgccagaaa accatgaacc aggatcgtgc catggcaatc ctgaatgatc    3060
   tgcgcaaaca gtaaaagctt ggcgtaatca tggtcatagc tgtttcctgt gtgaaattgt    3120
   tatccgctca caattccaca caacatacga gccggaagca taaagtgtaa agcctggggt    3180
   gcctaatgag tgagctaact cacattaatt gcgttgcgct cactgcccgc tttccagtcg    3240
   ggaaacctgt cgtgccagct gcattaatga atcggccaac gcgcggggag aggcggtttg    3300
   cgtattgggc gctcttccgc ttcctcgctc actgactcgc tgcgctcggt cgttcggctg    3360
   cggcgagcgg tatcagctca ctcaaaggcg gtaatacggt tatccacaga atcaggggat    3420
   aacgcaggaa agaacatgtg agcaaaaggc cagcaaaagg ccaggaaccg taaaaaggcc    3480
   gcgttgctgg cgtttttcca taggctccgc ccccctgacg agcatcacaa aaatcgacgc    3540
   tcaagtcaga ggtggcgaaa cccgacagga ctataaagat accaggcgtt tccccctgga    3600
   agctccctcg tgcgctctcc tgttccgacc ctgccgctta ccggatacct gtccgccttt    3660
   ctcccttcgg gaagcgtggc gctttctcat agctcacgct gtaggtatct cagttcggtg    3720
   taggtcgttc gctccaagct gggctgtgtg cacgaacccc ccgttcagcc cgaccgctgc    3780
   gccttatccg gtaactatcg tcttgagtcc aacccggtaa gacacgactt atcgccactg    3840
   gcagcagcca ctggtaacag gattagcaga gcgaggtatg taggcggtgc tacagagttc    3900
   ttgaagtggt ggcctaacta cggctacact agaagaacag tatttggtat ctgcgctctg    3960
   ctgaagccag ttaccttcgg aaaaagagtt ggtagctctt gatccggcaa acaaaccacc    4020
   gctggtagcg gtggtttttt tgtttgcaag cagcagatta cgcgcagaaa aaaaggatct    4080
   caagaagatc ctttgatctt ttctacgggg tctgacgctc agtggaacga aaactcacgt    4140
   taagggattt tggtcatgag attatcaaaa aggatcttca cctagatcct tttaaattaa    4200
   aaatgaagtt ttaaatcaat ctaaagtata tatgagtaaa cttggtctga cagttaccaa    4260
   tgcttaatca gtgaggcacc tatctcagcg atctgtctat ttcgttcatc catagttgcc    4320
   tgactccccg tcgtgtagat aactacgata cgggagggct taccatctgg ccccagtgct    4380
   gcaatgatac cgcgagaccc acgctcaccg gctccagatt tatcagcaat aaaccagcca    4440
   gccggaaggg ccgagcgcag aagtggtcct gcaactttat ccgcctccat ccagtctatt    4500
   aattgttgcc gggaagctag agtaagtagt tcgccagtta atagtttgcg caacgttgtt    4560
   gccattgcta caggcatcgt ggtgtcacgc tcgtcgtttg gtatggcttc attcagctcc    4620
   ggttcccaac gatcaaggcg agttacatga tcccccatgt tgtgcaaaaa agcggttagc    4680
   tccttcggtc ctccgatcgt tgtcagaagt aagttggccg cagtgttatc actcatggtt    4740
   atggcagcac tgcataattc tcttactgtc atgccatccg taagatgctt ttctgtgact    4800
   ggtgagtact caaccaagtc attctgagaa tagtgtatgc ggcgaccgag ttgctcttgc    4860
   ccggcgtcaa tacgggataa taccgcgcca catagcagaa ctttaaaagt gctcatcatt    4920
   ggaaaacgtt cttcggggcg aaaactctca aggatcttac cgctgttgag atccagttcg    4980
   atgtaaccca ctcgtgcacc caactgatct tcagcatctt ttactttcac cagcgtttct    5040
   gggtgagcaa aaacaggaag gcaaaatgcc gcaaaaaagg gaataagggc gacacggaaa    5100
   tgttgaatac tcatactctt cctttttcaa tattattgaa gcatttatca gggttattgt    5160
   ctcatgagcg gatacatatt tgaatgtatt tagaaaaata aacaaatagg ggttccgcgc    5220
   acatttcccc gaaaagtgcc acctgacgtc taagaaacca ttattatcat gacattaacc    5280
   tataaaaata ggcgtatcac gaggcccttt cgtc                                5314
<210> 4
   <211> 4054
   <212> DNA
   <213> Artificial Sequence
   <223> pUC57-EFmvaS
   <400> 4
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgcg gtgtgaaata ccgcacagat gcgtaaggag aaaataccgc atcaggcgcc     240
   attcgccatt caggctgcgc aactgttggg aagggcgatc ggtgcgggcc tcttcgctat     300
   tacgccagct ggcgaaaggg ggatgtgctg caaggcgatt aagttgggta acgccagggt     360
   tttcccagtc acgacgttgt aaaacgacgg ccagtgaatt cgagctcggt accttgacaa     420
   ttaatcatcc ggctcgtata atgtgtggaa ttgtgagcgg ataacaattt cacacaggaa     480
   acagcgccgc tgagaaaaag cgaagcggca ctgctcttta acaatttatc agacaatctg     540
   tgtgggcact cgaccggaat tatcgattaa ctttattatt aaaaattaaa gaggtatata     600
   ttaatgtatc gattaaataa ggaggaataa accatggatc cgagctcagg aggtaaaaaa     660
   acatgaccat tggtatcgat aaaattagct ttttcgtgcc gccgtattac atcgatatga     720
   cggcgctggc cgaagcacgt aacgttgatc cgggcaaatt tcatattggc atcggtcagg     780
   atcagatggc ggtgaacccg atttctcagg atatcgttac cttcgcggcc aatgcagcgg     840
   aagcaattct gacgaaagaa gataaagaag cgattgatat ggtgatcgtt ggcaccgaaa     900
   gctctatcga tgaaagtaaa gccgcagcgg tggttctgca ccgtctgatg ggcattcagc     960
   cgtttgcgcg cagcttcgaa atcaaagaag cctgctatgg cgcgaccgcc ggtctgcaac    1020
   tggccaaaaa ccatgtggca ctgcacccgg ataaaaaagt tctggtggtt gccgcagata    1080
   ttgcgaaata cggtctgaat agcggcggtg aaccgaccca gggtgcaggt gccgtggcaa    1140
   tgctggttgc atctgaaccg cgtattctgg cgctgaaaga agataacgtg atgctgaccc    1200
   aggatatcta tgatttttgg cgtccgaccg gtcatccgta cccgatggtg gatggcccgc    1260
   tgagtaatga aacctatatt cagagcttcg cccaggtttg ggatgaacat aaaaaacgta    1320
   cgggtctgga ttttgcggat tatgatgcac tggcgttcca cattccgtac accaaaatgg    1380
   gcaaaaaagc gctgctggcc aaaatcagcg atcagacgga agccgaacag gaacgtattc    1440
   tggcacgcta tgaagaaagc atcgtgtact ctcgtcgcgt tggcaacctg tataccggtt    1500
   ctctgtacct gggcctgatt agtctgctgg aaaacgcgac cacgctgacg gccggcaatc    1560
   agatcggtct gttttcttat ggcagtggtg ccgtggcaga atttttcacc ggtgaactgg    1620
   ttgccggcta ccagaaccat ctgcaaaaag aaacccacct ggccctgctg gataatcgca    1680
   cggaactgtc tattgcagaa tatgaagcaa tgtttgcgga aaccctggat acggatatcg    1740
   atcagaccct ggaagatgaa ctgaaatata gtattagcgc gatcaacaat acggtgcgta    1800
   gttaccgcaa ttaaaagctt ggcgtaatca tggtcatagc tgtttcctgt gtgaaattgt    1860
   tatccgctca caattccaca caacatacga gccggaagca taaagtgtaa agcctggggt    1920
   gcctaatgag tgagctaact cacattaatt gcgttgcgct cactgcccgc tttccagtcg    1980
   ggaaacctgt cgtgccagct gcattaatga atcggccaac gcgcggggag aggcggtttg    2040
   cgtattgggc gctcttccgc ttcctcgctc actgactcgc tgcgctcggt cgttcggctg    2100
   cggcgagcgg tatcagctca ctcaaaggcg gtaatacggt tatccacaga atcaggggat    2160
   aacgcaggaa agaacatgtg agcaaaaggc cagcaaaagg ccaggaaccg taaaaaggcc    2220
   gcgttgctgg cgtttttcca taggctccgc ccccctgacg agcatcacaa aaatcgacgc    2280
   tcaagtcaga ggtggcgaaa cccgacagga ctataaagat accaggcgtt tccccctgga    2340
   agctccctcg tgcgctctcc tgttccgacc ctgccgctta ccggatacct gtccgccttt    2400
   ctcccttcgg gaagcgtggc gctttctcat agctcacgct gtaggtatct cagttcggtg    2460
   taggtcgttc gctccaagct gggctgtgtg cacgaacccc ccgttcagcc cgaccgctgc    2520
   gccttatccg gtaactatcg tcttgagtcc aacccggtaa gacacgactt atcgccactg    2580
   gcagcagcca ctggtaacag gattagcaga gcgaggtatg taggcggtgc tacagagttc    2640
   ttgaagtggt ggcctaacta cggctacact agaagaacag tatttggtat ctgcgctctg    2700
   ctgaagccag ttaccttcgg aaaaagagtt ggtagctctt gatccggcaa acaaaccacc    2760
   gctggtagcg gtggtttttt tgtttgcaag cagcagatta cgcgcagaaa aaaaggatct    2820
   caagaagatc ctttgatctt ttctacgggg tctgacgctc agtggaacga aaactcacgt    2880
   taagggattt tggtcatgag attatcaaaa aggatcttca cctagatcct tttaaattaa    2940
   aaatgaagtt ttaaatcaat ctaaagtata tatgagtaaa cttggtctga cagttaccaa    3000
   tgcttaatca gtgaggcacc tatctcagcg atctgtctat ttcgttcatc catagttgcc    3060
   tgactccccg tcgtgtagat aactacgata cgggagggct taccatctgg ccccagtgct    3120
   gcaatgatac cgcgagaccc acgctcaccg gctccagatt tatcagcaat aaaccagcca    3180
   gccggaaggg ccgagcgcag aagtggtcct gcaactttat ccgcctccat ccagtctatt    3240
   aattgttgcc gggaagctag agtaagtagt tcgccagtta atagtttgcg caacgttgtt    3300
   gccattgcta caggcatcgt ggtgtcacgc tcgtcgtttg gtatggcttc attcagctcc    3360
   ggttcccaac gatcaaggcg agttacatga tcccccatgt tgtgcaaaaa agcggttagc    3420
   tccttcggtc ctccgatcgt tgtcagaagt aagttggccg cagtgttatc actcatggtt    3480
   atggcagcac tgcataattc tcttactgtc atgccatccg taagatgctt ttctgtgact    3540
   ggtgagtact caaccaagtc attctgagaa tagtgtatgc ggcgaccgag ttgctcttgc    3600
   ccggcgtcaa tacgggataa taccgcgcca catagcagaa ctttaaaagt gctcatcatt      3660
   ggaaaacgtt cttcggggcg aaaactctca aggatcttac cgctgttgag atccagttcg      3720
   atgtaaccca ctcgtgcacc caactgatct tcagcatctt ttactttcac cagcgtttct      3780
   gggtgagcaa aaacaggaag gcaaaatgcc gcaaaaaagg gaataagggc gacacggaaa      3840
   tgttgaatac tcatactctt cctttttcaa tattattgaa gcatttatca gggttattgt      3900
   ctcatgagcg gatacatatt tgaatgtatt tagaaaaata aacaaatagg ggttccgcgc      3960
   acatttcccc gaaaagtgcc acctgacgtc taagaaacca ttattatcat gacattaacc      4020
   tataaaaata ggcgtatcac gaggcccttt cgtc                                   4054
<210> 5
   <211> 38
   <212> DNA
   <213> Artificial Sequence
   <223> Primer to amplify trc promoter (Ptrc)
   <400> 5
   gaattcgagc tcggtaccct tgacaattaa tcatccgg 38
<210> 6
   <211> 45
   <212> DNA
   <213> Artificial Sequence
   <223> Primer to amplify trc promoter (Ptrc)
   <400> 6
   aaccacggtt ttcatgtttt tttacctcct gagctcggat ccatg 45
<210> 7
   <211> 45
   <212> DNA
   <213> Artificial Sequence
   <223> Primer to amplify mvaE
   <400> 7
   catggatccg agctcaggag gtaaaaaaac atgaaaaccg tggtt 45
<210> 8
   <211> 55
   <212> DNA
   <213> Artificial Sequence
   <223> Primer to amplify mvaE, mvaS
<400> 8
   ttatcgatac caatggtcat gtttttttac ctcctttact gtttgcgcag atcat 55
<210> 9
   <211> 55
   <212> DNA
   <213> Artificial Sequence
   <223> Primer to amplify mvaS
<400> 9
   atgatctgcg caaacagtaa aggaggtaaa aaaacatgac cattggtatc gataa 55
<210> 10
   <211> 40
   <212> DNA
   <213> Artificial Sequence
   <223> Primer to amplify tryptophane terminator (Ttrp)
<400> 10
   cagcggaact ggcggctccc ttaattgcgg taactacgca 40
<210> 11
   <211> 40
   <212> DNA
   <213> Artificial Sequence
   <223> Primer to amplify tryptophane terminator (Ttrp)
   <400> 11
   tgcgtagtta ccgcaattaa gggagccgcc agttccgctg 40
<210> 12
   <211> 38
   <212> DNA
   <213> Artificial Sequence
   <223> Primer
<400> 12
   gtcgactcta gaggatccct aatgagaatt agtcaaat 38
<210> 13
   <211> 8053
   <212> DNA
   <213> Artificial Sequence
   <223> pMW-Ptrc-mvaES-Ttrp
   <400> 13
   gacagtaaga cgggtaagcc tgttgatgat accgctgcct tactgggtgc attagccagt      60
   ctgaatgacc tgtcacggga taatccgaag tggtcagact ggaaaatcag agggcaggaa     120
   ctgcagaaca gcaaaaagtc agatagcacc acatagcaga cccgccataa aacgccctga     180
   gaagcccgtg acgggctttt cttgtattat gggtagtttc cttgcatgaa tccataaaag     240
   gcgcctgtag tgccatttac ccccattcac tgccagagcc gtgagcgcag cgaactgaat     300
   gtcacgaaaa agacagcgac tcaggtgcct gatggtcgga gacaaaagga atattcagcg     360
   atttgcccga gcttgcgagg gtgctactta agcctttagg gttttaaggt ctgttttgta     420
   gaggagcaaa cagcgtttgc gacatccttt tgtaatactg cggaactgac taaagtagtg     480
   agttatacac agggctggga tctattcttt ttatcttttt ttattctttc tttattctat     540
   aaattataac cacttgaata taaacaaaaa aaacacacaa aggtctagcg gaatttacag     600
   agggtctagc agaatttaca agttttccag caaaggtcta gcagaattta cagataccca     660
   caactcaaag gaaaaggact agtaattatc attgactagc ccatctcaat tggtatagtg     720
   attaaaatca cctagaccaa ttgagatgta tgtctgaatt agttgttttc aaagcaaatg     780
   aactagcgat tagtcgctat gacttaacgg agcatgaaac caagctaatt ttatgctgtg     840
   tggcactact caaccccacg attgaaaacc ctacaaggaa agaacggacg gtatcgttca     900
   cttataacca atacgctcag atgatgaaca tcagtaggga aaatgcttat ggtgtattag     960
   ctaaagcaac cagagagctg atgacgagaa ctgtggaaat caggaatcct ttggttaaag    1020
   gctttgagat tttccagtgg acaaactatg ccaagttctc aagcgaaaaa ttagaattag    1080
   tttttagtga agagatattg ccttatcttt tccagttaaa aaaattcata aaatataatc    1140
   tggaacatgt taagtctttt gaaaacaaat actctatgag gatttatgag tggttattaa    1200
   aagaactaac acaaaagaaa actcacaagg caaatataga gattagcctt gatgaattta    1260
   agttcatgtt aatgcttgaa aataactacc atgagtttaa aaggcttaac caatgggttt    1320
   tgaaaccaat aagtaaagat ttaaacactt acagcaatat gaaattggtg gttgataagc    1380
   gaggccgccc gactgatacg ttgattttcc aagttgaact agatagacaa atggatctcg    1440
   taaccgaact tgagaacaac cagataaaaa tgaatggtga caaaatacca acaaccatta    1500
   catcagattc ctacctacat aacggactaa gaaaaacact acacgatgct ttaactgcaa    1560
   aaattcagct caccagtttt gaggcaaaat ttttgagtga catgcaaagt aagcatgatc    1620
   tcaatggttc gttctcatgg ctcacgcaaa aacaacgaac cacactagag aacatactgg    1680
   ctaaatacgg aaggatctga ggttcttatg gctcttgtat ctatcagtga agcatcaaga    1740
   ctaacaaaca aaagtagaac aactgttcac cgttacatat caaagggaaa actgtccata    1800
   tgcacagatg aaaacggtgt aaaaaagata gatacatcag agcttttacg agtttttggt    1860
   gcattcaaag ctgttcacca tgaacagatc gacaatgtaa cagatgaaca gcatgtaaca    1920
   cctaatagaa caggtgaaac cagtaaaaca aagcaactag aacatgaaat tgaacacctg    1980
   agacaacttg ttacagctca acagtcacac atagacagcc tgaaacaggc gatgctgctt    2040
   atcgaatcaa agctgccgac aacacgggag ccagtgacgc ctcccgtggg gaaaaaatca    2100
   tggcaattct ggaagaaata gcgccattcg ccattcaggc tgcgcaactg ttgggaaggg    2160
   cgatcggtgc gggcctcttc gctattacgc cagctggcga aagggggatg tgctgcaagg    2220
   cgattaagtt gggtaacgcc agggttttcc cagtcacgac gttgtaaaac gacggccagt    2280
   gaattcgagc tcggtaccct tgacaattaa tcatccggct cgtataatgt gtggaattgt    2340
   gagcggataa caatttcaca caggaaacag cgccgctgag aaaaagcgaa gcggcactgc    2400
   tctttaacaa tttatcagac aatctgtgtg ggcactcgac cggaattatc gattaacttt    2460
   attattaaaa attaaagagg tatatattaa tgtatcgatt aaataaggag gaataaacca    2520
   tggatccgag ctcaggaggt aaaaaaacat gaaaaccgtg gttattatcg atgcgctgcg    2580
   cacgccgatt ggtaaatata aaggcagcct gtctcaagtg agcgccgttg atctgggtac    2640
   gcatgtgacc acgcagctgc tgaaacgtca cagcaccatc tctgaagaaa ttgatcaggt    2700
   gatctttggt aacgttctgc aagccggtaa tggtcagaat ccggcacgtc agattgcaat    2760
   caacagtggc ctgagccatg aaattccggc gatgaccgtg aatgaagttt gcggtagcgg    2820
   catgaaagcg gttattctgg ccaaacagct gatccagctg ggtgaagcgg aagtgctgat    2880
   tgccggcggt atcgaaaaca tgagtcaggc accgaaactg caacgtttta attatgaaac    2940
   cgaaagctac gatgccccgt tcagctctat gatgtatgat ggcctgaccg atgcatttag    3000
   cggtcaggcg atgggcctga cggcagaaaa cgtggcggaa aaataccatg ttacccgcga    3060
   agaacaggat cagttttctg ttcacagtca gctgaaagcg gcccaggccc aggcagaagg    3120
   tattttcgcc gatgaaatcg caccgctgga agtgtctggt acgctggttg aaaaagatga    3180
   aggcattcgt ccgaatagta gcgtggaaaa actgggcacc ctgaaaacgg tgttcaaaga    3240
   agatggcacc gttacggcgg gcaatgcaag caccatcaat gatggtgcga gtgccctgat    3300
   tatcgcgagc caggaatatg cagaagcgca tggcctgccg tacctggcca ttatccgcga    3360
   ttctgtggaa gttggtattg atccggcata tatgggcatt agtccgatca aagcgattca    3420
   gaaactgctg gcccgtaacc agctgaccac cgaagaaatt gatctgtacg aaatcaatga    3480
   agcgtttgca gcgaccagta ttgtggttca gcgcgaactg gccctgccgg aagaaaaagt    3540
   taacatttat ggcggtggca tcagcctggg tcacgcaatt ggtgccaccg gtgcacgtct    3600
   gctgaccagt ctgagctatc agctgaatca gaaagagaaa aaatacggtg tggcaagcct    3660
   gtgtattggt ggcggtctgg gtctggccat gctgctggaa cgtccgcagc agaagaaaaa    3720
   ctctcgtttt taccagatga gtccggaaga acgtctggcc agtctgctga acgaaggcca    3780
   gattagcgca gataccaaaa aagaattcga aaatacggca ctgtctagtc agatcgcgaa    3840
   ccatatgatt gaaaatcaga tcagcgaaac cgaagtgccg atgggtgttg gcctgcacct    3900
   gaccgtggat gaaacggatt atctggttcc gatggcgacg gaagaaccga gcgttattgc    3960
   cgcactgtct aacggtgcaa aatcgcgcca gggctttaaa accgtgaatc agcagcgtct    4020
   gatgcgcggc cagattgtgt tctacgatgt tgcggatccg gaaagcctga tcgataaact    4080
   gcaagtgcgc gaagccgaag tttttcagca ggcagaactg agctatccgt ctattgtgaa    4140
   acgtggcggt ggcctgcgcg atctgcaata ccgtaccttt gatgaaagtt tcgtgagcgt    4200
   tgatttcctg gtggatgtta aagatgccat gggtgcaaac atcgtgaatg cgatgctgga    4260
   aggcgttgcc gaactgtttc gtgaatggtt cgcggaacag aaaatcctgt tttctatcct    4320
   gagtaactac gcgaccgaaa gcgtggttac catgaaaacg gccattcctg tgagccgcct    4380
   gtctaaaggt agtaatggcc gtgaaattgc ggaaaaaatc gttctggcga gccgctatgc    4440
   ctctctggat ccgtaccgtg ccgtgaccca taacaaaggt attatgaatg gcatcgaagc    4500
   agtggttctg gcgaccggta acgatacccg tgccgtgtct gcaagttgcc atgcattcgc    4560
   agttaaagaa ggtcgttatc agggcctgac cagctggacg ctggatggtg aacagctgat    4620
   cggcgaaatt tctgtgccgc tggccctggc aaccgtgggt ggcgcgacga aagttctgcc    4680
   gaaaagccag gcggccgcag atctgctggc ggtgaccgat gcaaaagaac tgtctcgcgt    4740
   ggttgcggcc gttggtctgg cacagaatct ggcagcgctg cgtgcgctgg tgtctgaagg    4800
   tattcagaaa ggccacatgg cactgcaagc ccgtagtctg gccatgaccg tgggtgcaac    4860
   gggcaaagaa gtggaagcag ttgcgcagca gctgaaacgc cagaaaacca tgaaccagga    4920
   tcgtgccatg gcaatcctga atgatctgcg caaacagtaa aggaggtaaa aaaacatgac    4980
   cattggtatc gataaaatta gctttttcgt gccgccgtat tacatcgata tgacggcgct    5040
   ggccgaagca cgtaacgttg atccgggcaa atttcatatt ggcatcggtc aggatcagat    5100
   ggcggtgaac ccgatttctc aggatatcgt taccttcgcg gccaatgcag cggaagcaat    5160
   tctgacgaaa gaagataaag aagcgattga tatggtgatc gttggcaccg aaagctctat    5220
   cgatgaaagt aaagccgcag cggtggttct gcaccgtctg atgggcattc agccgtttgc    5280
   gcgcagcttc gaaatcaaag aagcctgcta tggcgcgacc gccggtctgc aactggccaa    5340
   aaaccatgtg gcactgcacc cggataaaaa agttctggtg gttgccgcag atattgcgaa    5400
   atacggtctg aatagcggcg gtgaaccgac ccagggtgca ggtgccgtgg caatgctggt    5460
   tgcatctgaa ccgcgtattc tggcgctgaa agaagataac gtgatgctga cccaggatat    5520
   ctatgatttt tggcgtccga ccggtcatcc gtacccgatg gtggatggcc cgctgagtaa      5580
   tgaaacctat attcagagct tcgcccaggt ttgggatgaa cataaaaaac gtacgggtct      5640
   ggattttgcg gattatgatg cactggcgtt ccacattccg tacaccaaaa tgggcaaaaa      5700
   agcgctgctg gccaaaatca gcgatcagac ggaagccgaa caggaacgta ttctggcacg      5760
   ctatgaagaa agcatcgtgt actctcgtcg cgttggcaac ctgtataccg gttctctgta      5820
   cctgggcctg attagtctgc tggaaaacgc gaccacgctg acggccggca atcagatcgg      5880
   tctgttttct tatggcagtg gtgccgtggc agaatttttc accggtgaac tggttgccgg      5940
   ctaccagaac catctgcaaa aagaaaccca cctggccctg ctggataatc gcacggaact      6000
   gtctattgca gaatatgaag caatgtttgc ggaaaccctg gatacggata tcgatcagac      6060
   cctggaagat gaactgaaat atagtattag cgcgatcaac aatacggtgc gtagttaccg      6120
   caattaaggg agccgccagt tccgctggcg gcattttaac tttctttaat gaagccggaa      6180
   aaatcctaaa ttcatttaat atttatcttt ttaccgtttc gcttaccccg gtcgaacgtc      6240
   aacttacgtc atttttccgc ccaacagtaa tataatcaaa caaattaatc ccgcaacata      6300
   acaccagtaa aatcaataat tttctctaag tcacttattc ctcaggtaat tgttaatata      6360
   tccagaatgt tcctcaaaat atattttccc tctatcttct cgttgcgctt aatttgacta      6420
   attctcatta gggatcctct agagtcgacc tgcaggcatg caagcttggc gtaatcatgg      6480
   tcatagctgt ttcctgtgtg aaattgttat ccgctcacaa ttccacacaa catacgagcc      6540
   ggaagcataa agtgtaaagc ctggggtgcc taatgagtga gctaactcac attaattgcg      6600
   ttgcgctcac tgcccgcttt ccagtcggga aacctgtcgt gccagctgca ttaatgaatc      6660
   ggccaacgcg cggggagagg cggtttgcgt attgggcgct ttctcatagc tcacgctgta      6720
   ggtatctcag ttcggtgtag gtcgttcgct ccaagctggg ctgtgtgccg aaccccagag      6780
   tcccgctcag aagaactcgt caagaaggcg atagaaggcg atgcgctgcg aatcgggagc      6840
   ggcgataccg taaagcacga ggaagcggtc agcccattcg ccgccaagct cttcagcaat      6900
   atcacgggta gccaacgcta tgtcctgata gcggtccgcc acacccagcc ggccacagtc      6960
   gatgaatcca gaaaagcggc cattttccac catgatattc ggcaagcagg catcgccatg      7020
   ggtcacgacg agatcctcgc cgtcgggcat gcgcgccttg agcctggcga acagttcggc      7080
   tggcgcgagc ccctgatgct cttcgtccag atcatcctga tcgacaagac cggcttccat      7140
   ccgagtacgt gctcgctcga tgcgatgttt cgcttggtgg tcgaatgggc aggtagccgg      7200
   atcaagcgta tgcagccgcc gcattgcatc agccatgatg gatactttct cggcaggagc      7260
   aaggtgagat gacaggagat cctgccccgg cacttcgccc aatagcagcc agtcccttcc      7320
   cgcttcagtg ccaacgtcga gcacagctgc gcaaggaacg cccgtcgtgg ccagccacga      7380
   tagccgcgct gcctcgtcct gcagttcatt cagggcaccg gacaggtcgg tcttgacaaa      7440
   aagaaccggg cgcccctgcg ctgacagccg gaacacggcg gcatcagagc agccgattgt      7500
   ctgttgtgcc cagtcatagc cgaatagcct ctccacccaa gcggccggag aacctgcgtg      7560
   caatccatct tgttcaatca tgcgaaacga tcctcatcct gtctcttgat cactaccgca      7620
   ttaaagcata tcgatgataa gctgtcaaac atgagcgttc ttcggggcga aaactctcaa      7680
   ggatcttacc gctgttgaga tccagttcga tgtaacccac tcgtgcaccc aactgatctt      7740
   cagcatcttt tactttcacc agcgtttctg ggtgagcaaa aacaggaagg caaaatgccg      7800
   caaaaaaggg aataagggcg acacggaaat gttgaatact catactcttc ctttttcaat      7860
   attattgaag catttatcag ggttattgtc tcatgagcgg atacatattt gaatgtattt      7920
   agaaaaataa acaaataggg gttccgcgca catttccccg aaaagtgcca cctgacgtct      7980
   aagaaaccat tattatcatg acattaacct ataaaaatag gcgtatcacg aggccctttc      8040
   gtcttcaaga att                                                         8053
<210> 14
   <211> 40
   <212> DNA
   <213> Artificial Sequence
   <223> Primer to amplify pMW-Ptrc-mvaES-Ttrp
   <400> 14
   ctctaaggag gttataaaaa atgaaaaccg tggttattat 40
<210> 15
   <211> 40
   <212> DNA
   <213> Artificial Sequence
   <223> Primer to amplify pMW-Ptrc-mvaES-Ttrp
   <400> 15
   aatattgaaa aaggaagagt gggtaccgag ctcgaattca 40
<210> 16
   <211> 40
   <212> DNA
   <213> Artificial Sequence
   <223> Primer to amplify pKD46
   <400> 16
   tgaattcgag ctcggtaccc actcttcctt tttcaatatt 40
<210> 17
   <211> 40
   <212> DNA
   <213> Artificial Sequence
   <223> Primer to amplify pKD46
   <400> 17
   ataataacca cggttttcat tttttataac ctccttagag 40
<210> 18
   <211> 9180
   <212> DNA
   <213> Artificial Sequence
   <223> pMW-Para-mvaES-Ttrp
   <400> 18
   gacagtaaga cgggtaagcc tgttgatgat accgctgcct tactgggtgc attagccagt        60
   ctgaatgacc tgtcacggga taatccgaag tggtcagact ggaaaatcag agggcaggaa       120
   ctgcagaaca gcaaaaagtc agatagcacc acatagcaga cccgccataa aacgccctga       180
   gaagcccgtg acgggctttt cttgtattat gggtagtttc cttgcatgaa tccataaaag       240
   gcgcctgtag tgccatttac ccccattcac tgccagagcc gtgagcgcag cgaactgaat       300
   gtcacgaaaa agacagcgac tcaggtgcct gatggtcgga gacaaaagga atattcagcg       360
   atttgcccga gcttgcgagg gtgctactta agcctttagg gttttaaggt ctgttttgta       420
   gaggagcaaa cagcgtttgc gacatccttt tgtaatactg cggaactgac taaagtagtg       480
   agttatacac agggctggga tctattcttt ttatcttttt ttattctttc tttattctat       540
   aaattataac cacttgaata taaacaaaaa aaacacacaa aggtctagcg gaatttacag       600
   agggtctagc agaatttaca agttttccag caaaggtcta gcagaattta cagataccca       660
   caactcaaag gaaaaggact agtaattatc attgactagc ccatctcaat tggtatagtg       720
   attaaaatca cctagaccaa ttgagatgta tgtctgaatt agttgttttc aaagcaaatg       780
   aactagcgat tagtcgctat gacttaacgg agcatgaaac caagctaatt ttatgctgtg       840
   tggcactact caaccccacg attgaaaacc ctacaaggaa agaacggacg gtatcgttca       900
   cttataacca atacgctcag atgatgaaca tcagtaggga aaatgcttat ggtgtattag       960
   ctaaagcaac cagagagctg atgacgagaa ctgtggaaat caggaatcct ttggttaaag      1020
   gctttgagat tttccagtgg acaaactatg ccaagttctc aagcgaaaaa ttagaattag      1080
   tttttagtga agagatattg ccttatcttt tccagttaaa aaaattcata aaatataatc      1140
   tggaacatgt taagtctttt gaaaacaaat actctatgag gatttatgag tggttattaa      1200
   aagaactaac acaaaagaaa actcacaagg caaatataga gattagcctt gatgaattta      1260
   agttcatgtt aatgcttgaa aataactacc atgagtttaa aaggcttaac caatgggttt      1320
   tgaaaccaat aagtaaagat ttaaacactt acagcaatat gaaattggtg gttgataagc      1380
   gaggccgccc gactgatacg ttgattttcc aagttgaact agatagacaa atggatctcg      1440
   taaccgaact tgagaacaac cagataaaaa tgaatggtga caaaatacca acaaccatta      1500
   catcagattc ctacctacat aacggactaa gaaaaacact acacgatgct ttaactgcaa      1560
   aaattcagct caccagtttt gaggcaaaat ttttgagtga catgcaaagt aagcatgatc      1620
   tcaatggttc gttctcatgg ctcacgcaaa aacaacgaac cacactagag aacatactgg      1680
   ctaaatacgg aaggatctga ggttcttatg gctcttgtat ctatcagtga agcatcaaga      1740
   ctaacaaaca aaagtagaac aactgttcac cgttacatat caaagggaaa actgtccata      1800
   tgcacagatg aaaacggtgt aaaaaagata gatacatcag agcttttacg agtttttggt      1860
   gcattcaaag ctgttcacca tgaacagatc gacaatgtaa cagatgaaca gcatgtaaca      1920
   cctaatagaa caggtgaaac cagtaaaaca aagcaactag aacatgaaat tgaacacctg      1980
   agacaacttg ttacagctca acagtcacac atagacagcc tgaaacaggc gatgctgctt      2040
   atcgaatcaa agctgccgac aacacgggag ccagtgacgc ctcccgtggg gaaaaaatca    2100
   tggcaattct ggaagaaata gcgccattcg ccattcaggc tgcgcaactg ttgggaaggg    2160
   cgatcggtgc gggcctcttc gctattacgc cagctggcga aagggggatg tgctgcaagg    2220
   cgattaagtt gggtaacgcc agggttttcc cagtcacgac gttgtaaaac gacggccagt    2280
   gaattcgagc tcggtaccca ctcttccttt ttcaatatta ttgaagcatt tatcagggtt    2340
   attgtctcat gagcggatac atatttgaat gtatttagaa aaataaacaa ataggggttc    2400
   cgcgcacatt tccccgaaaa gtgccacctg catcgattta ttatgacaac ttgacggcta    2460
   catcattcac tttttcttca caaccggcac ggaactcgct cgggctggcc ccggtgcatt    2520
   ttttaaatac ccgcgagaag tagagttgat cgtcaaaacc aacattgcga ccgacggtgg    2580
   cgataggcat ccgggtggtg ctcaaaagca gcttcgcctg gctgatacgt tggtcctcgc    2640
   gccagcttaa gacgctaatc cctaactgct ggcggaaaag atgtgacaga cgcgacggcg    2700
   acaagcaaac atgctgtgcg acgctggcga tatcaaaatt gctgtctgcc aggtgatcgc    2760
   tgatgtactg acaagcctcg cgtacccgat tatccatcgg tggatggagc gactcgttaa    2820
   tcgcttccat gcgccgcagt aacaattgct caagcagatt tatcgccagc agctccgaat    2880
   agcgcccttc cccttgcccg gcgttaatga tttgcccaaa caggtcgctg aaatgcggct    2940
   ggtgcgcttc atccgggcga aagaaccccg tattggcaaa tattgacggc cagttaagcc    3000
   attcatgcca gtaggcgcgc ggacgaaagt aaacccactg gtgataccat tcgcgagcct    3060
   ccggatgacg accgtagtga tgaatctctc ctggcgggaa cagcaaaata tcacccggtc    3120
   ggcaaacaaa ttctcgtccc tgatttttca ccaccccctg accgcgaatg gtgagattga    3180
   gaatataacc tttcattccc agcggtcggt cgataaaaaa atcgagataa ccgttggcct    3240
   caatcggcgt taaacccgcc accagatggg cattaaacga gtatcccggc agcaggggat    3300
   cattttgcgc ttcagccata cttttcatac tcccgccatt cagagaagaa accaattgtc    3360
   catattgcat cagacattgc cgtcactgcg tcttttactg gctcttctcg ctaaccaaac    3420
   cggtaacccc gcttattaaa agcattctgt aacaaagcgg gaccaaagcc atgacaaaaa    3480
   cgcgtaacaa aagtgtctat aatcacggca gaaaagtcca cattgattat ttgcacggcg    3540
   tcacactttg ctatgccata gcatttttat ccataagatt agcggatcct acctgacgct    3600
   ttttatcgca actctctact gtttctccat acccgttttt ttgggaattc gagctctaag    3660
   gaggttataa aaaatgaaaa ccgtggttat tatcgatgcg ctgcgcacgc cgattggtaa    3720
   atataaaggc agcctgtctc aagtgagcgc cgttgatctg ggtacgcatg tgaccacgca    3780
   gctgctgaaa cgtcacagca ccatctctga agaaattgat caggtgatct ttggtaacgt    3840
   tctgcaagcc ggtaatggtc agaatccggc acgtcagatt gcaatcaaca gtggcctgag    3900
   ccatgaaatt ccggcgatga ccgtgaatga agtttgcggt agcggcatga aagcggttat    3960
   tctggccaaa cagctgatcc agctgggtga agcggaagtg ctgattgccg gcggtatcga    4020
   aaacatgagt caggcaccga aactgcaacg ttttaattat gaaaccgaaa gctacgatgc    4080
   cccgttcagc tctatgatgt atgatggcct gaccgatgca tttagcggtc aggcgatggg    4140
   cctgacggca gaaaacgtgg cggaaaaata ccatgttacc cgcgaagaac aggatcagtt    4200
   ttctgttcac agtcagctga aagcggccca ggcccaggca gaaggtattt tcgccgatga    4260
   aatcgcaccg ctggaagtgt ctggtacgct ggttgaaaaa gatgaaggca ttcgtccgaa    4320
   tagtagcgtg gaaaaactgg gcaccctgaa aacggtgttc aaagaagatg gcaccgttac    4380
   ggcgggcaat gcaagcacca tcaatgatgg tgcgagtgcc ctgattatcg cgagccagga    4440
   atatgcagaa gcgcatggcc tgccgtacct ggccattatc cgcgattctg tggaagttgg    4500
   tattgatccg gcatatatgg gcattagtcc gatcaaagcg attcagaaac tgctggcccg    4560
   taaccagctg accaccgaag aaattgatct gtacgaaatc aatgaagcgt ttgcagcgac    4620
   cagtattgtg gttcagcgcg aactggccct gccggaagaa aaagttaaca tttatggcgg    4680
   tggcatcagc ctgggtcacg caattggtgc caccggtgca cgtctgctga ccagtctgag    4740
   ctatcagctg aatcagaaag agaaaaaata cggtgtggca agcctgtgta ttggtggcgg    4800
   tctgggtctg gccatgctgc tggaacgtcc gcagcagaag aaaaactctc gtttttacca    4860
   gatgagtccg gaagaacgtc tggccagtct gctgaacgaa ggccagatta gcgcagatac    4920
   caaaaaagaa ttcgaaaata cggcactgtc tagtcagatc gcgaaccata tgattgaaaa    4980
   tcagatcagc gaaaccgaag tgccgatggg tgttggcctg cacctgaccg tggatgaaac    5040
   ggattatctg gttccgatgg cgacggaaga accgagcgtt attgccgcac tgtctaacgg    5100
   tgcaaaaatc gcgcagggct ttaaaaccgt gaatcagcag cgtctgatgc gcggccagat    5160
   tgtgttctac gatgttgcgg atccggaaag cctgatcgat aaactgcaag tgcgcgaagc    5220
   cgaagttttt cagcaggcag aactgagcta tccgtctatt gtgaaacgtg gcggtggcct    5280
   gcgcgatctg caataccgta cctttgatga aagtttcgtg agcgttgatt tcctggtgga    5340
   tgttaaagat gccatgggtg caaacatcgt gaatgcgatg ctggaaggcg ttgccgaact    5400
   gtttcgtgaa tggttcgcgg aacagaaaat cctgttttct atcctgagta actacgcgac    5460
   cgaaagcgtg gttaccatga aaacggccat tcctgtgagc cgcctgtcta aaggtagtaa     5520
   tggccgtgaa attgcggaaa aaatcgttct ggcgagccgc tatgcctctc tggatccgta    5580
   ccgtgccgtg acccataaca aaggtattat gaatggcatc gaagcagtgg ttctggcgac    5640
   cggtaacgat acccgtgccg tgtctgcaag ttgccatgca ttcgcagtta aagaaggtcg    5700
   ttatcagggc ctgaccagct ggacgctgga tggtgaacag ctgatcggcg aaatttctgt    5760
   gccgctggcc ctggcaaccg tgggtggcgc gacgaaagtt ctgccgaaaa gccaggcggc    5820
   cgcagatctg ctggcggtga ccgatgcaaa agaactgtct cgcgtggttg cggccgttgg 5880
   tctggcacag aatctggcag cgctgcgtgc gctggtgtct gaaggtattc agaaaggcca 5940
   catggcactg caagcccgta gtctggccat gaccgtgggt gcaacgggca aagaagtgga 6000
   agcagttgcg cagcagctga aacgccagaa aaccatgaac caggatcgtg ccatggcaat 6060
   cctgaatgat ctgcgcaaac agtaaaggag gtaaaaaaac atgaccattg gtatcgataa 6120
   aattagcttt ttcgtgccgc cgtattacat cgatatgacg gcgctggccg aagcacgtaa 6180
   cgttgatccg ggcaaatttc atattggcat cggtcaggat cagatggcgg tgaacccgat 6240
   ttctcaggat atcgttacct tcgcggccaa tgcagcggaa gcaattctga cgaaagaaga 6300
   taaagaagcg attgatatgg tgatcgttgg caccgaaagc tctatcgatg aaagtaaagc 6360
   cgcagcggtg gttctgcacc gtctgatggg cattcagccg tttgcgcgca gcttcgaaat 6420
   caaagaagcc tgctatggcg cgaccgccgg tctgcaactg gccaaaaacc atgtggcact 6480
   gcacccggat aaaaaagttc tggtggttgc cgcagatatt gcgaaatacg gtctgaatag 6540
   cggcggtgaa ccgacccagg gtgcaggtgc cgtggcaatg ctggttgcat ctgaaccgcg 6600
   tattctggcg ctgaaagaag ataacgtgat gctgacccag gatatctatg atttttggcg 6660
   tccgaccggt catccgtacc cgatggtgga tggcccgctg agtaatgaaa cctatattca 6720
   gagcttcgcc caggtttggg atgaacataa aaaacgtacg ggtctggatt ttgcggatta 6780
   tgatgcactg gcgttccaca ttccgtacac caaaatgggc aaaaaagcgc tgctggccaa 6840
   aatcagcgat cagacggaag ccgaacagga acgtattctg gcacgctatg aagaaagcat 6900
   cgtgtactct cgtcgcgttg gcaacctgta taccggttct ctgtacctgg gcctgattag 6960
   tctgctggaa aacgcgacca cgctgacggc cggcaatcag atcggtctgt tttcttatgg 7020
   cagtggtgcc gtggcagaat ttttcaccgg tgaactggtt gccggctacc agaaccatct 7080
   gcaaaaagaa acccacctgg ccctgctgga taatcgcacg gaactgtcta ttgcagaata 7140
   tgaagcaatg tttgcggaaa ccctggatac ggatatcgat cagaccctgg aagatgaact 7200
   gaaatatagt attagcgcga tcaacaatac ggtgcgtagt taccgcaatt aagggagccg 7260
   ccagttccgc tggcggcatt ttaactttct ttaatgaagc cggaaaaatc ctaaattcat 7320
   ttaatattta tctttttacc gtttcgctta ccccggtcga acgtcaactt acgtcatttt 7380
   tccgcccaac agtaatataa tcaaacaaat taatcccgca acataacacc agtaaaatca 7440
   ataattttct ctaagtcact tattcctcag gtaattgtta atatatccag aatgttcctc 7500
   aaaatatatt ttccctctat cttctcgttg cgcttaattt gactaattct cattaggggg 7560
   atcctctaga gtcgacctgc aggcatgcaa gcttggcgta atcatggtca tagctgtttc 7620
   ctgtgtgaaa ttgttatccg ctcacaattc cacacaacat acgagccgga agcataaagt 7680
   gtaaagcctg gggtgcctaa tgagtgagct aactcacatt aattgcgttg cgctcactgc 7740
   ccgctttcca gtcgggaaac ctgtcgtgcc agctgcatta atgaatcggc caacgcgcgg 7800
   ggagaggcgg tttgcgtatt gggcgctttc tcatagctca cgctgtaggt atctcagttc 7860
   ggtgtaggtc gttcgctcca agctgggctg tgtgccgaac cccagagtcc cgctcagaag 7920
   aactcgtcaa gaaggcgata gaaggcgatg cgctgcgaat cgggagcggc gataccgtaa 7980
   agcacgagga agcggtcagc ccattcgccg ccaagctctt cagcaatatc acgggtagcc 8040
   aacgctatgt cctgatagcg gtccgccaca cccagccggc cacagtcgat gaatccagaa 8100
   aagcggccat tttccaccat gatattcggc aagcaggcat cgccatgggt cacgacgaga 8160
   tcctcgccgt cgggcatgcg cgccttgagc ctggcgaaca gttcggctgg cgcgagcccc 8220
   tgatgctctt cgtccagatc atcctgatcg acaagaccgg cttccatccg agtacgtgct 8280
   cgctcgatgc gatgtttcgc ttggtggtcg aatgggcagg tagccggatc aagcgtatgc 8340
   agccgccgca ttgcatcagc catgatggat actttctcgg caggagcaag gtgagatgac 8400
   aggagatcct gccccggcac ttcgcccaat agcagccagt cccttcccgc ttcagtgcca 8460
   acgtcgagca cagctgcgca aggaacgccc gtcgtggcca gccacgatag ccgcgctgcc 8520
   tcgtcctgca gttcattcag ggcaccggac aggtcggtct tgacaaaaag aaccgggcgc 8580
   ccctgcgctg acagccggaa cacggcggca tcagagcagc cgattgtctg ttgtgcccag 8640
   tcatagccga atagcctctc cacccaagcg gccggagaac ctgcgtgcaa tccatcttgt 8700
   tcaatcatgc gaaacgatcc tcatcctgtc tcttgatcac taccgcatta aagcatatcg 8760
   atgataagct gtcaaacatg agcgttcttc ggggcgaaaa ctctcaagga tcttaccgct 8820
   gttgagatcc agttcgatgt aacccactcg tgcacccaac tgatcttcag catcttttac 8880
   tttcaccagc gtttctgggt gagcaaaaac aggaaggcaa aatgccgcaa aaaagggaat 8940
   aagggcgaca cggaaatgtt gaatactcat actcttcctt tttcaatatt attgaagcat 9000
   ttatcagggt tattgtctca tgagcggata catatttgaa tgtatttaga aaaataaaca 9060
   aataggggtt ccgcgcacat ttccccgaaa agtgccacct gacgtctaag aaaccattat 9120
   tatcatgaca ttaacctata aaaataggcg tatcacgagg ccctttcgtc ttcaagaatt 9180
<210> 19
   <211> 33
   <212> DNA
   <213> Artificial Sequence
<223> n67
<400> 19
   tgcgaagacg tcctcgtgaa gaaggtgttg ctg 33
<210> 20
   <211> 36
   <212> DNA
   <213> Artificial Sequence
   <223> n68
   <400> 20
   tgcgaagggc cccgttgtgt ctcaaaatct ctgatg 36
<210> 21
   <211> 3471
   <212> DNA
   <213> Artificial Sequence
   <223> Sequence of the chemically synthesized DNA fragment retaining
   artificial KDyI operon with optimized codons
   <400> 21
   gcatgcagga ggtatgaatg tcagagttgc gtgccttcag tgccccaggg aaagcgttac      60
   tcgctggtgg atatttagtt ttagatacaa aatatgaagc atttgtagtc ggattatcgg     120
   cacgtatgca cgctgtagcc catccttacg gttcattgca agggtctgat aagtttgaag     180
   tgcgtgtgaa aagtaaacaa tttaaagatg gggagtggct gtaccatata agtcctaaaa      240
   gtggcttcat tcctgtttcg ataggcggat ctaagaaccc tttcattgaa aaagttatcg     300
   ctaacgtatt tagctacttt aaacctaaca tggacgacta ctgcaatcgt aacttgttcg     360
   ttattgatat tttctctgat gatgcctacc attctcagga ggatagcgtt accgaacatc      420
   gtggcaaccg ccgtttgagt tttcattcgc accgtattga agaagttccc aaaacagggc     480
   tgggctcctc ggcaggttta gtcacagttt taactacagc tttggcctcc ttttttgtat     540
   cggacctgga aaataatgta gacaaatatc gtgaagttat tcataattta gcacaagttg      600
   ctcattgtca agctcagggt aaaattggaa gcgggtttga tgtagcggcg gcagcatatg     660
   gatctatccg ttatcgccgt ttcccacccg cattaatctc taatttgcca gatattggaa     720
   gtgctactta cggcagtaaa ctggcgcatt tggttgatga agaagactgg aatattacga     780
   ttaaaagtaa ccatttacct tcgggattaa ctttatggat gggcgatatt aagaatggtt     840
   cagaaacagt aaaactggtc cagaaggtaa aaaattggta tgattcgcat atgccagaaa     900
   gcctcaaaat atatacagaa ctcgatcatg caaattctcg ttttatggat ggactctcta     960
   aactcgatcg cttacacgag actcatgacg attacagcga tcagatattt gagtctcttg    1020
   agcgtaatga ctgtacctgt caaaagtatc ctgaaatcac agaagttcgt gatgcagttg    1080
   ccacaattcg tcgttccttt cgtaaaataa ctaaagaatc tggtgccgat atcgaacctc    1140
   ccgtacaaac tagcttattg gatgattgcc agaccttaaa aggagttctt acttgcttaa    1200
   tacctggtgc tggtggttat gacgccattg cagtgattac taagcaagat gttgatcttc    1260
   gtgctcaaac cgctaatgac aaacgttttt ctaaggttca atggctggat gtaactcagg    1320
   ctgactgggg tgttcgtaaa gaaaaagatc cggaaactta tcttgataaa taactgcaga    1380
   ggaggtatga atgaccgttt acacagcatc cgttaccgca cccgtcaaca tcgcaaccct    1440
   taagtattgg gggaaacgtg acacgaagtt gaatctgccc accaattcgt ccatatcagt    1500
   gactttatcg caagatgacc tccgtacgtt gacctctgcg gctactgcac ctgagtttga    1560
   acgcgacact ttgtggttaa atggagaacc acacagcatc gacaatgaac gtactcaaaa    1620
   ttgtctgcgc gacctccgcc aattacgtaa ggaaatggaa tcgaaggacg cctcattgcc    1680
   cacattatct caatggaaac tccacattgt ctccgaaaat aactttccta cagcagctgg    1740
   tttagcttcc tccgctgctg gctttgctgc attggtctct gcaattgcta agttatacca    1800
   attaccacag tcaacttcag aaatatctcg tatagcacgt aaggggtctg gttcagcttg    1860
   tcgttcgttg tttggcggat acgtggcctg ggaaatggga aaagctgaag atggtcatga    1920
   ttccatggca gtacaaatcg cagacagctc tgactggcct cagatgaaag catgtgtcct    1980
   tgttgtcagc gatattaaaa aggatgtgag ttccactcag ggtatgcaat tgaccgtggc    2040
   aacctccgaa ctctttaaag aacgtattga acatgtcgta ccaaagcgtt ttgaagtcat    2100
   gcgtaaagcc attgttgaaa aagatttcgc cacctttgca aaggaaacaa tgatggattc    2160
   caactctttc catgccacat gtttggactc tttccctcca atattctaca tgaatgacac    2220
   ttccaagcgt atcatcagtt ggtgccacac cattaatcag ttttacggag aaacaatcgt    2280
   tgcatacacg tttgatgcag gtccaaatgc tgtgttgtac tacttagctg aaaatgagtc    2340
   gaaactcttt gcatttatct ataaattgtt tggctctgtt cctggatggg acaagaaatt    2400
   tactactgag cagcttgagg ctttcaacca tcaatttgaa tcatctaact ttactgcacg      2460
   tgaattggat cttgagttgc aaaaggatgt tgcccgtgtg attttaactc aagtcggttc      2520
   aggcccacaa gaaacaaacg aatctttgat tgacgcaaag actggtctcc caaaggaata      2580
   aggatccagg aggtatgaat gactgccgac aacaatagta tgccccatgg tgcagtatct      2640
   agttacgcca aattagtgca aaaccaaaca cctgaagaca ttttggaaga gtttcctgaa      2700
   attattccat tacaacaacg tcctaatacc cgctctagtg agacgtcaaa tgacgaaagc      2760
   ggagaaacat gtttttctgg tcatgatgag gagcaaatta agttaatgaa tgaaaattgt      2820
   attgttttgg attgggacga taatgctatt ggtgccggca ccaagaaagt ttgtcattta      2880
   atggaaaata ttgaaaaggg tttacttcat cgtgcattct ccgtctttat tttcaatgaa      2940
   caaggtgaat tacttttaca acaacgtgcc actgaaaaaa taactttccc tgatctttgg      3000
   actaacacat gctgctctca tccactttgt attgatgacg aattaggttt gaagggtaag      3060
   ctcgacgata agattaaggg cgctattact gcggcggtgc gtaaactcga tcatgaatta      3120
   ggtattccag aagatgaaac taagacacgt ggtaagtttc actttttaaa ccgtatccat      3180
   tacatggcac caagcaatga accatggggt gaacatgaaa ttgattacat cctcttttat      3240
   aagatcaacg ctaaagaaaa cttgactgtc aacccaaacg tcaatgaagt tcgtgacttc      3300
   aaatgggttt caccaaatga tttgaaaact atgtttgctg acccaagtta caagtttacg      3360
   ccttggttta agattatttg cgagaattac ttattcaact ggtgggagca attagatgac      3420
   ctttctgaag tggaaaatga ccgtcaaatt catcgtatgc tctaaggtac c               3471
<210> 22
   <211> 900
   <212> DNA
   <213> Artificial Sequence
   <223> Methanocella paludicola
   <400> 22
   atgacgatgt gttcagcccc cggtaaggtc tttttattcg gcgagcatgc ggtcgtttac        60
   ggcaagcgcg cgatcgcatg cgccatcgac ctgcggacca ccgtggaggt atcccgtaaa       120
   agccgtggcg tccacattca ctcggccttc aaggacgagc cggataagaa cctgtatatc       180
   aagacggccg ttaaaaggat gcagaagtgt gcggatatcc ggaacgtgaa catcgctgtt       240
   tcctcgagga tccccgtggc ttcgggcctg ggctcctcgg ctgccgtgac cgtcgccacc       300
   atcggcgcct tgaacgagga gttcagcgcc ggcttgagta aaaaagacat cgcgtatatg       360
   gcctaccaga cagagctgga agtgcaggga gcggcgagcc cgaccgatac gttcgtgtcc       420
   accatgggcg ggaccgttgt cgtgcccgac atgaggacac tgccgcctat cacctgcggc       480
   atcgtggtcg gccacaccgg catatcaaaa tccacttcac gcatggtatc ccgggtgaga       540
   acgctcaaag aaaaataccc ggacgtcatc gacggcatca tggactcgat cggggatatc       600
   tccgcgcggg gcgaggattt aataaaacag aacgactatc gttccatagg cgagctcatg       660
   aacgtcaacc aggggctgct ggacgccctg ggcatcacca tacccgagct ctcactccag       720
   atatatgccg cacgccagca cggcgcctac ggcgccaaga tcaccggcgc gggcggaggg       780
   ggatgcatgg tggccatatg cgatgacaaa aattgtaaag agatcgcgac cgccatcggg       840
   cgctcatacg gcgacagctt catcagcaag ccaacggcgg agggaatatt catccaatga       900
<210> 23
   <211> 70
   <212> DNA
   <213> Artificial Sequence
   <223> ampH-attL-phi80
   <400> 23
   atgcgcactc cttacgtact ggctctactg gtttctttgc gaaaggtcat ttttcctgaa        60
   tatgctcaca                                                               70
<210> 24
   <211> 64
   <212> DNA
   <213> Artificial Sequence
   <223> ampH-attR-phi80
   <400> 24
   ttaaggaatc gcctggacca tcatcggcga gccgttctga cgtttgttga cagctggtcc 60
   aatg 64
<210> 25
   <211> 68
   <212> DNA
   <213> Artificial Sequence
   <223> DampC-phL
   <400> 25
   ctgatgaact gtcacctgaa tgagtgctga tgaaaatata gaaaggtcat ttttcctgaa 60
   tatgctca 68
<210> 26
   <211> 64
   <212> DNA
   <213> Artificial Sequence
   <223> DampC-phR
   <400> 26
   attcgccagc ataacgatgc cgctgttgag ctgaggaaca cgtttgttga cagctggtcc 60
   aatg 64
<210> 27
   <211> 18
   <212> DNA
   <213> Artificial Sequence
   <223> ampH-t1
   <400> 27
   gcgaagccct ctccgttg 18
<210> 28
   <211> 21
   <212> DNA
   <213> Artificial Sequence
   <223> ampH-t2
   <400> 28
   agccagtcag cctcatcagc g 21
<210> 29
   <211> 21
   <212> DNA
   <213> Artificial Sequence
   <223> ampC-t1
<400> 29
   gattcccact tcaccgagcc g 21
<210> 30
   <211> 21
   <212> DNA
   <213> Artificial Sequence
   <223> ampC-t2
   <400> 30
   ggcaggtatg gtgctctgac g 21
<210> 31
   <211> 64
   <212> DNA
   <213> Artificial Sequence
   <223> crtE-attRphi80
   <400> 31
   atgacggtct gcgcaaaaaa acacgttcat ctcactcgcg cgtttgttga cagctggtcc 60
   aatg 64
<210> 32
   <211> 68
   <212> DNA
   <213> Artificial Sequence
   <223> crtZ-attLphi80
   <400> 32
   atgttgtgga tttggaatgc cctgatcgtt ttcgttaccg gaaaggtcat ttttcctgaa 60
   tatgctca 68
<210> 33
   <211> 21
   <212> DNA
   <213> Artificial Sequence
   <223> crtz-test
   <400> 33
   ccgtgtggtt ctgaaagccg a 21
<210> 34
   <211> 21
   <212> DNA
   <213> Artificial Sequence
   <223> crtE-test
   <400> 34
   cgttgccgta aatgtatccg t 21

## Description

### TECHNICAL FIELD

This disclosure relates to a method for producing polyisoprene.

### BACKGROUND

A rubber product such as tire is requested of excellent breaking resistance, wear resistance, crack growth resistance, etc., and thus a rubber component having excellent elasticity is necessarily used as its raw material Known as the rubber component is a natural rubber collected from a rubber tree, which mainly contains polyisoprene with a high molecular weight.

Recently, natural rubber is in high demand, while on the other hand, along with the reduction of rubber tree resource, the price of natural rubber is rising. Therefore, preparation of a polyisoprene with properties identical to a polyisoprene of rubber tree derived natural rubber is being studied widely.

Known as the polyisoprene instead of the polyisoprene of natural rubber are synthesized polyisoprene prepared with a chemical method such as distillation of petroleum, and fermented polyisoprene prepared with a method using microbes (see PTL1 to PTL3).

### CITATION LIST

### Patent Literature

PTL1: JP2011505841A
PTL2: JP2011518564A
PTL3: JP2013514375A

### SUMMARY

### (Technical Problem)

However, an isoprene-containing composition using an isoprene prepared with the aforementioned conventional methods, particularly in the case with existence of impurities inhibiting the polymerization of the isoprene, there was a risk of deterioration of the catalytic activity, which disables production of a polyisoprene with sufficiently high molecular weight.

Then, this disclosure aims to provide a method for producing polyisoprene, which, even in the case with existence of impurities other than isoprene inhibiting the polymerization of isoprene, is capable of controlling its type and amount, and controlling the type and among of the polymerization catalyst, to thereby produce a polyisoprene with a high molecular weight as compared to the case of polymerizing a highly purified isoprene.

### (Solution to Problem)

The gist of the present disclosure is as follows.

The method for producing polyisoprene of this disclosure is a method for producing polyisoprene comprising: polymerizing isoprene by using an isoprene-containing composition containing isoprene and an oxygen-containing neutral compound, and a polymerization catalyst composition containing a rare earth element compound and an organometallic compound, wherein:
a compounding amount of the organometallic compound is 0.6 to 3.0 parts by mass per 100 parts by mass of the isoprene-containing composition.

In the method for producing polyisoprene of this disclosure, it is preferable that a compounding amount of the oxygen-containing neutral compound is 0.05 to 1.0 parts by mass per 100 parts by mass of the isoprene-containing composition.

Moreover, in the method for producing polyisoprene of this disclosure, the isoprene-containing composition may be prepared via a fermentation method.

Further, in the method for producing polyisoprene of this disclosure, it is preferable that the oxygen-containing neutral compound is selected from the group consisting of an ether, an ester and a ketone.

Further, in the method for producing polyisoprene of this disclosure, it is preferable that a number-average molecular weight (Mn) of the polyisoprene is 1,000,000 or more.

Further, in the method for producing polyisoprene of this disclosure, it is preferable that the number-average molecular weight (Mn) of the polyisoprene is 1,300,000 or more.

Further, in the method for producing polyisoprene of this disclosure, it is preferable that a molecular weight distribution (Mw/Mn) of the polyisoprene is 2.5 or less.

Further, in the method for producing polyisoprene of this disclosure, it is preferable that a cis-1,4-bond content of the polyisoprene is 98% or more.

Further, in the method for producing polyisoprene of this disclosure, it is preferable that the rare earth element compound is a rare earth element-containing compound or a reaction product of the rare earth element-containing compound and a Lewis base, the rare earth element-containing compound or reaction product having metal-nitrogen bond (M-N bond), and the rare earth element-containing compound is a compound containing scandium, yttrium or a lanthanoid element selected from elements of atomic numbers 57 to 71.

Further, in the method for producing polyisoprene of this disclosure, it is preferable that the organometallic compound is a compound represented with
the following formula (S2)

YR⁴ₐR⁵_{b}R⁶_{c}... (S2)

where Y is a metallic element selected from the group consisting of Group 1, Group 2, Group 12 and Group 13; R⁴ and R⁵ are C1 to C10 hydrocarbon group or hydrogen atom, and may be either identical or different; R⁶ is a C1 to C10 hydrocarbon group; R⁶ may be either identical to or different from the R⁴ or R⁵; if Y is a Group 1 metallic element, a is 1, and b, c are 0; if Y is a Group 2 metallic element or a Group 12 metallic element, a and b are 1, and c is 0; and if Y is a Group 13 metallic element, a, b and c are 1; or
the following formula (S3)

AlR⁷R⁸R⁹ ... (S3)

where R⁷ and R⁸ are C1 to C10 hydrocarbon group or hydrogen atom, and may be either identical or different; R⁹ is a C1 to C10 hydrocarbon group; and R⁹ may be either identical to or different from the R⁷ or R⁸.

Further, in the method for producing polyisoprene of this disclosure, it is preferable that the polymerization catalyst composition is at least one compound selected from the group consisting of an aluminoxane compound, a halogen compound, an ionic compound, and a compound capable of serving as an anionic ligand.

### (Advantageous Effect)

According to the method for producing polyisoprene of this disclosure, it is possible to produce a polyisoprene having a comparatively high molecular weight, and to thereby improve the produced synthesized polyisoprene.

### BRIEF DESCRIPTION OF THE DRAWINGS

FIG. 1 illustrates a pAH162-P^{ara}-mvaES plasmid constructed in the examples;
FIG. 2A illustrates a pAH162-*λ*attL-Tc^{R}-*λ*attR integrative vector, and FIG. 2B illustrates the pAH162-*λ*attL-Km^{R}-*λ*attR integrative vector constructed in the examples;
FIG. 3 illustrates a pAH162-P_{tac} integrative expression vector used in the examples;
FIG. 4 illustrates a KDyI operon with a chemically synthesized codon optimized;
FIG. 5A illustrates a plasmid pAH162-Tc-P_{tac}-KDyI constructed in the examples for holding a KDyI operon with an optimized codon, and FIG. 5B illustrates a plasmid pAH162-Km-P_{tac}-KDyI constructed in the examples for holding a KDyI operon with an optimized codon;
FIG. 6 illustrates an integrative plasmid pAH162-P_{tac}-mvk constructed in the examples for holding an mvk gene derived from M. paludicola;
FIG. 7A illustrates a modified genome ΔampC::attBₚₕᵢ₈₀ constructed in the examples; FIG. 7B illustrates a modified genome ΔampH::attBₚₕᵢ₈₀ constructed in the examples; and FIG. 7C illustrates a modified genome Δcrt::attBₚₕᵢ₈₀ constructed in the examples;
FIG. 8A illustrates a modified genome Δcrt::pAH162-P_{tac}-mvk constructed in the examples, and FIG. 8B illustrates a modified genome Δcrt::P_{tac}-mvk constructed in the examples;
FIG. 9A illustrates a modified genome AampH::pAH162-Km-P_{tac}-KDyl constructed in the examples, and FIG. 9B illustrates a modified genome ΔampC::pAH162-Km-P_{tac}-KDyI constructed in the examples;
FIG. 10A illustrates a modified genome ΔampH::pAH162-Pₐᵣₐ-mvaES constructed in the examples, and FIG. 10B illustrates a modified genome ΔampC::pAH162-Pₐᵣₐ-mvaES constructed in the examples; and
FIG. 11 illustrates an outline of an isoprene recovery apparatus used in the examples.

### DETAILED DESCRIPTION

### (Method for producing polyisoprene)

An embodiment for the method for producing polyisoprene of this disclosure is described in detail hereinafter.

The method for producing polyisoprene of an example of this disclosure (referred to as "the production method of the example" as well hereinafter) is a method for polymerizing isoprene by using: an isoprene-containing composition containing isoprene and an oxygen-containing neutral compound, and a polymerization catalyst composition containing a rare earth element compound and an organometallic compound.

### -Isoprene-containing composition-

The isoprene-containing composition used in the method for producing polyisoprene of the examples of this disclosure (referred to as "the isoprene-containing composition" as well hereinafter) contains isoprene and an oxygen-containing neutral compound.

Here, the oxygen-containing neutral compound is considered as coordinated to a central metal of a metallic catalyst used in the polymerization of isoprene, thereby improving the catalytic activity. According to the method for producing polyisoprene using this isoprene-containing composition, it is possible to produce a polyisoprene with a comparatively high molecular weight.

### --Isoprene--

The isoprene contained in the isoprene-containing composition is not specifically limited, and may be either an isoprene obtainable with a chemical method (synthesized isoprene) or an isoprene prepared with a method using microbes (fermented isoprene).

The synthesized isoprene may be obtained as a C₅ fraction in fractional distillation of petroleum.

The fermented isoprene may be obtained by, for example, the fermentation mentioned below, and in this case, the product of fermentation method may be used directly as the isoprene-containing composition.

It is preferable that the synthesized isoprene and the fermented isoprene are purified before used in the polymerization reaction, where the necessity is higher as for the fermented isoprene.

Examples of the method for purifying the fermented isoprene include column chromatography, distillation, etc., and in particular, from the viewpoint of simple convenience and economy, column chromatography is preferable.

In the case of column chromatography, examples of a filler of the column (solid) include activated alumina, silica gel, molecular sieve, reduced copper, etc., and in particular, activated alumina is preferable, and examples of an elute (liquid) include hydrocarbons, etc., and in particular, hexane is preferable.

Here, from the viewpoint of sufficiently removing impurities inhibiting the polymerization of the isoprene, a weight ratio of the filler with respect to isoprene is preferably 0.1 to 2.0, more preferably 0.5 to 1.0. A purification time is preferably 2 to 8 hours, more preferably 4 to 6 hours.

### --Oxygen-containing neutral compound--

Examples of the oxygen-containing neutral compound include ether, ester, ketone, etc.

Specific examples of the ester include ethyl acetate, methyl acetate, etc., and specific examples of the ketone include methyl vinyl ketone, methyl ethyl ketone (2-butanone), diacetyl(2,3-butanedione), methyl isobutyl ketone, etc. In particular, ethyl acetate and 3-methylfuran are preferable.

By selecting the aforementioned compounds as the oxygen-containing neutral compound, it is possible to improve the effect of the method for producing polyisoprene to produce a polyisoprene having a comparatively high molecular weight.

Note that the isoprene-containing composition may contain components other than the aforementioned isoprene and oxygen-containing neutral compound.

Moreover, the isoprene-containing composition refers to one without containing a solvent, and may be, for example, one consisting of merely the aforementioned isoprene and oxygen-containing neutral compound.

According to the method for producing polyisoprene using the aforementioned isoprene-containing composition, it is possible to produce a polyisoprene having a comparatively high molecular weight with existence of an oxygen-containing neutral compound other than isoprene. Thereby, it is possible to improve the strength of the produced synthesized polyisoprene.

Here, the isoprene-containing composition is an isoprene-containing composition of which a weight of isoprene is 98.5 mass% or more. From the viewpoint of sufficiently obtaining the effect of this disclosure, this ratio is preferably 98.8 mass% or more, further preferably 99.0 mass% or more.

Here, from the viewpoint of sufficiently obtaining the aforementioned effect of the method for manufacturing polyisoprene, i.e., the capacity of producing a polyisoprene having a comparatively high molecular weight, the compounding amount of the oxygen-containing neutral compound is 0.05 parts by mass or more, preferably 0.07 parts by mass or more, particularly preferably 0.1 parts by mass or more per 100 parts by mass of isoprene. Moreover, from the viewpoint of reducing the risk that the the oxygen-containing neutral compound deteriorates the catalytic activity in contrary, the compounding amount of the oxygen-containing neutral compound is preferably 1.0 part by mass or less, more preferably 0.7 parts by mass or less, particularly preferably 0.5 parts by mass or less.

The fermentation method for producing the aforementioned fermented isoprene is described hereinafter.

In the fermentation method, a gene encoding isoprene synthase is transduced into a host cell, thereby transforming the host cell, to culture this transformant and produce isoprene.

In the method according to the example according to the fermentation method, from the viewpoint of increasing the production volume of isoprene, in addition to the gene encoding isoprene synthase, it is preferable to produce the transformant by further transducing genes encoding enzymes involved in a mevalonic acid (MVA) pathway and/or a methyl erythritol phosphate (MEP) pathway involved in synthesis of dimethylallyl diphosphate (DMAPP), which is a substrate of isoprene synthase.

Further, in the method according to the example, from the viewpoint of increasing the production volume of isoprene, it is preferable to produce the transformant by further transducing a gene encoding an isopentenyl-diphosphate delta isomerase having a capacity of converting isopentenyl diphosphate (IPP) into dimethylallyl diphosphate (DMAPP).

The enzyme expressed in the transformant in the fermentation method may be either identical to or different from the species of host cell.

As an example of this disclosure, it is preferable to use Pantoea ananatis as a host cell, one derived from Mucuna as an isoprene synthase, one derived from Saccharomyces cerevisiae, Enterococcus faecalis or Metanocella pardicola as an enzyme involved in the mevalonic acid pathway, and one derived from Saccharomyces cerevisiae as an isopentenyl-diphosphate delta isomerase. According to this combination, isoprene may be produced at a high efficiency.

### [Host]

A host used in the method according to the example may be any cell without being limited, as long as containing genes encoding enzymes involved in a mevalonic acid (MVA) pathway and/or a methyl erythritol phosphate (MEP) pathway involved in synthesis of dimethylallyl diphosphate (DMAPP), which is a substrate of isoprene synthase. The host is preferably a bacterium or a fungus.

The bacterium may be either a gram-positive bacterium or a gram-negative bacterium.

Examples of the gram-positive bacterium include bacteria belonging to the genera Bacillus, Listeria, Staphylococcus, Streptococcus, Enterococcus, Clostridium, Corynebacterium and Streptomyces. Bacteria belonging to the genera Bacillus and Corynebacterium are preferable.

Examples of the bacteria belonging to the genus Bacillus may include Bacillus subtilis, Bacillus anthracis, and Bacillus cereus. Bacillus subtilis is more preferable. Examples of the bacteria belonging to the genus Corynebacterium may include Corynebacterium glutamicum, Corynebacterium efficiens, and Corynebacterium callunae. Corynebacterium glutamicum is more preferable.

Examples of the gram-negative bacterium may include bacteria belonging to the genera Escherichia, Pantoea, Salmonella, Vivrio, Serratia, and Enterobacter. The bacteria belonging to the genera Escherichia, Pantoea and Enterobacter are preferable.

Escherichia coli is preferable as the bacteria belonging to the genus Escherichia.

Exmaples for a strain of Escherichia coli (E. coli) include well-known ones in the art, for example, DH5α, JM109, etc.

Examples of the bacteria belonging to the genus Pantoea may include Pantoea ananatis, Pantoea stewartii, Pantoea agglomerans, and Pantoea citrea. Pantoea ananatis and Pantoea citrea are preferable. Strains exemplified in EP0952221, which is incorporated herein by reference in its entirety, may be used as the bacteria belonging to the genus Pantoea. Examples of representative strains of the bacteria belonging to the genus Pantoea may include Pantoea ananatis AJ13355 strain (FERM BP-6614) and Pantoea ananatis AJ13356 strain (FERM BP-6615), both of which are disclosed in EP0952221, which is incorporated herein by reference in its entirety, and Pantoea ananatis SC17(0) strain. Pantoea ananatis SC17(0) was deposited to Russian National Collection of Industrial Microorganisms (VKPM), GNII Genetika (address: Russia, 117545 Moscow, 1 Dorozhny proezd. 1) as of Sep. 21, 2005, with the deposit number of VKPM B-9246.

Examples of the bacteria belonging to the genus Enterobacter may include Enterobacter agglomerans and Enterobacter aerogenes. Enterobacter aerogenes is preferable. The bacterial strains exemplified in EP0952221, which is incorporated herein by reference in its entirety, may be used as the bacteria belonging to the genus Enterobacter. Examples of representative strains of the bacteria belonging to the genus Enterobacter may include Enterobacter agglomerans ATCC12287 strain, Enterobacter aerogenes ATCC13048 strain, Enterobacter aerogenes NBRC12010 strain (Biotechnol. Bioeng., 2007 Mar. 27; 98(2): 340-348, which is incorporated herein by reference in its entirety), and Enterobacter aerogenes AJ110637 (FERM BP-10955). The Enterobacter aerogenes AJ110637 strain was deposited to International Patent Organism Depositary (IPOD), National Institute of Advanced Industrial Science and Technology (AIST) (Chuo No. 6, Higashi 1-1-1, Tsukuba City, Ibaraki Pref., JP, Postal code 305-8566) as of Aug. 22, 2007, with the deposit number of FERM P-21348 and was transferred to the international deposition based on Budapest Treaty on Mar. 13, 2008, and the receipt number FERM BP-10955 was given thereto.

Examples of the fungus may include microorganisms belonging to the genera Saccharomyces, Schizosaccharomyces, Yarrowia, Trichoderma, Aspergillus, Fusarium, and Mucor. The microorganisms belonging to the genera Saccharomyces, Schizosaccharomyces, Yarrowia, or Trichoderma are preferable.

Examples of the microorganisms belonging to the genus Saccharomyces may include Saccharomyces carlsbergensis, Saccharomyces cerevisiae, Saccharomyces diastaticus, Saccharomyces douglasii, Saccharomyces kluyveri, Saccharomyces norbensis, and Saccharomyces oviformis. Saccharomyces cerevisiae is preferable.

Schizosaccharomyces pombe is preferable as a microorganism belonging to the genus Schizosaccharomyces.

Yarrowia lypolytica is preferable as a microorganism belonging to the genus Yarrowia.

Examples of the microorganisms belonging to the genus Trichoderma may include Trichoderma harzianum, Trichoderma koningii, Trichoderma longibrachiatum, Trichoderma reesei, and Trichoderma viride. Trichoderma reesei is preferable.

Other than the bacteria and the fungi mentioned above, examples of the host include an insect cell, an animal cell, a plant cell, a bacterial cell, etc.

Note that such host may be used alone or in a combination of two or more.

### [Isoprene synthase]

Examples of the isoprene synthase used in the method according to the example include isoprene synthase derived from kudzu (Pueraria montana) (IspK), isoprene synthase derived from poplar, isoprene synthase derived from Mucuna (Mucuna pruriens) (IspM) (US20140113344A1), isoprene synthase derived from willow (Salix), isoprene synthase derived from false acacia (Robinia pseudoacacia), isoprene synthase derived from wisteria (Wisterria), isoprene synthase derived from eucalyptus (Eucalyptus globulus), isoprene synthase derived from tea tree (Melaleuca alterniflora), etc. (see, e.g., Evolution 67 (4), 1026-1040 (2013) which is incorporated herein by reference in its entirety). In a preferable embodiment, the isoprene synthase may be derived from kudzu; in another preferable embodiment, the isoprene synthase may be derived from poplar; and in still another preferable embodiment, the isoprene synthase may be derived from Mucuna.

Note that such isoprene synthase may be used alone or in a combination of two or more.

### [Enzyme involved in mevalonic acid (MVA) pathway]

Examples of the enzymes involved in the mevalonate (MVA) pathway may include mevalonate kinase (EC: 2.7.1.36; example 1, Erg12p, ACCESSION ID NP_013935; example 2, AT5G27450, ACCESSION ID NP_001190411), phosphomevalonate kinase (EC: 2.7.4.2; example 1, Erg8p, ACCESSION ID NP_013947; example 2, AT1G31910, ACCESSION ID NP_001185124), diphosphomevalonate decarboxylase (EC: 4.1.1.33; example 1, Mvd1p, ACCESSION ID NP_014441; example 2, AT2G38700, ACCESSION ID NP_181404; example 3, AT3G54250, ACCESSION ID NP_566995), acetyl-CoA-C-acetyltransferase (EC: 2.3.1.9; example 1, Erg10p, ACCESSION ID NP_015297; example 2, AT5G47720, ACCESSION ID NP_001032028; example 3, AT5G48230, ACCESSION ID NP_568694), hydroxymethylglutaryl-CoA synthase (EC: 2.3.3.10; example 1, Erg13p, ACCESSION ID NP _013580; example 2, AT4G11820, ACCESSION ID NP_192919; example 3, MvaS, ACCESSION ID AAG02438), hydroxymethylglutaryl-CoA reductase (EC: 1.1.1.34; example 1, Hmg2p, ACCESSION ID NP_013555; example 2, Hmglp, ACCESSION ID NP_{_}013636; example 3, AT1G76490, ACCESSION ID NP_177775; example 4, AT2G17370, ACCESSION ID NP_179329, EC: 1.1.1.88, example, MvaA, ACCESSION ID P13702), and acetyl-CoA-C-acetyltransferase/hydroxymethylglutaryl-CoA reductase (EC: 2.3.1.9/1.1.1.34, example, MvaE, ACCESSION ID AAG02439).

Examples of the enzymes involved in the mevalonate (MVA) pathway may include those expressed from genes derived from microorganisms belonging to the genus Methanosarcina such as Methanosarcina mazei, the genus Methanocella such as Methanocella paludicola, the genus Corynebacterium such as Corynebacterium variabile, the genus Methanosaeta such as Methanosaeta concilii, and the genus Nitrosopumilus such as Nitrosopumilus maritimus.

Note that such enzyme involved in the mevalonic acid (MVA) pathway may be used alone or in a combination of two or more.

### [Enzyme involved in the methylerythritol phosphate (MEP) pathway]

Examples of the enzymes involved in the methylerythritol phosphate (MEP) pathway may include 1-deoxy-D-xylulose-5-phosphate synthase (which synthesizes 1-deoxy-D-xylose-5-phosphate from a pyruvate and D-glyceraldehyde-3-phosphate) (EC: 2.2.1.7, example 1, Dxs, ACCESSION ID NP_414954; example 2, AT3G21500, ACCESSION ID NP_566686; example 3, AT4G15560, ACCESSION ID NP_193291; example 4, AT5G11380, ACCESSION ID NP_001078570), 1-deoxy-D-xylulose-5-phosphate reductoisomerase (EC: 1.1.1.267; example 1, Dxr, ACCESSION ID NP_414715; example 2, AT5G62790, ACCESSION ID NP_001190600), 4-diphosphocytidyl-2-C-methyl-D-erythritol synthase (EC: 2.7.7.60; example 1, IspD, ACCESSION ID NP_417227; example 2, AT2G02500, ACCESSION ID NP_565286), 4-diphosphocytidyl-2-C-methyl-D-erythritol kinase (EC: 2.7.1.148; example 1, IspE, ACCESSION ID NP_415726; example 2, AT2G26930, ACCESSION ID NP_180261), 2-C-methyl-D-erythritol-2,4-cyclodiphosphate synthase (EC: 4.6.1.12; example 1, IspF, ACCESSION ID NP_417226; example 2, AT1G63970, ACCESSION ID NP_564819), 1-hydroxy-2-methyl-2-(E)-butenyl-4-diphosphate synthase (EC: 1.17.7.1; example 1, IspG, ACCESSION ID NP_417010; example 2, AT5G60600, ACCESSION ID NP_001119467), and 4-hydroxy-3-methyl-2-butenyl diphosphate reductase (EC: 1.17.1.2; example 1, IspH, ACCESSION ID NP_414570; example 2, AT4G34350, ACCESSION ID NP_567965).

The MEP pathway is known as widely existing in prokaryotic microorganisms and plants, and enzymes derived from these creatures may be used (Eisenreich W et al., Biosynthesis of isoprenoids via the non-mevalonate pathway, Cell Mol Life Sci. 61, 1401-1426, 2004). Examples thereof include those expressed from genes derived from Escherichia coli, Pantoea ananatis, Corynebacterium glutamicum, creatures belonging to the genus Enterobacter, etc.

Note that the aforementioned enzymes involved in the methylerythritol phosphate (MEP) pathway may be used alone or in a combination of two or more.

### [Isopentenyl-diphosphate delta isomerase]

Examples of isopentenyl-diphosphate delta isomerase (isopentenyl-diphosphate isomerase) may include Idilp (ACCESSION ID NP_015208), AT3G02780 (ACCESSION ID NP_186927), AT5G16440 (ACCESSION ID NP_197148) and Idi (ACCESSION ID NP_417365).

In the fermentation method used in this disclosure, enzymes other than the aforementioned enzymes and proteins other than enzymes may be used as necessary.

As long as having the functions of the aforementioned enzymes or proteins, modified products of the aforementioned enzymes or proteins may be used, without being limited to the amino acid sequence of the aforementioned enzymes or proteins. Examples for the modified products include those having substitution, deletion, insertion, addition and the like of one or more amino acids.

Note that, although varying depending on the types of amino acid residual groups related to the substitution, deletion, insertion, addition and the like, and the position in the three-dimensional structure of the protein of the amino acid residual group, "one or more" refers to 1 to 10, preferably 1 to 5, more preferably 1 to 3, further more preferably 1 to 2.

Specific examples of those having substitution include those having conservative substitution.

"Conservative substitution" refers to substitution between amino acids having similar properties, and specifically refers to substitution between Phe, Trp or Tyr in the case of aromatic amino acids, substitution between Leu, Ile or Val in the case of hydrophobic amino acids, substitution between Gln and Asn in the case of polar amino acids, substitution between Lys, Arg or His in the case of basic amino acids, substitution between Asp and Glu in the case of acidic amino acids, and substitution between Ser and Thr in the case of amino acids having hydroxyl groups.

Specific examples of substitution treated as conservative substitution include substitution from Ala to Ser or Thr; substitution from Arg to Gln, His or Lys; substitution from Asn to Glu, Gln, Lys, His or Asp; substitution from Asp to Asn, Glu or Gln; substitution from Cys to Ser or Ala; substitution from Gln to Asn, Glu, Lys, His, Asp or Arg; substitution from Glu to Gly, Asn, Gln, Lys or Asp; substitution from Gly to Pro; substitution from His to Asn, Lys, Gln, Arg or Tyr; substitution from Ile to Leu, Met, Val or Phe; substitution from Leu to Ile, Met, Val or Phe; substitution from Lys to Asn, Glu, Gln, His or Arg; substitution from Met to Ile, Leu, Val or Phe; substitution from Phe to Trp, Tyr, Met, Ile or Leu; substitution from Ser to Thr or Ala; substitution from Thr to Ser or Ala; substitution from Trp to Phe or Tyr; substitution from Tyr to His, Phe or Trp; and substitution from Val to Met, Ile or Leu.

Base sequences (DNA sequences and RNA sequences) of the genes encoding the aforementioned enzymes or proteins are well known in the art.

Such base sequence is not limited to the aforementioned base sequences as long as having the functions of the enzyme or protein encoded in its base sequence, and modified products of the aforementioned base sequence may be used.

The modified products may be either obtained via naturally occurring mutantation or obtained via artificial mutantation (e.g., by transducing a variation from/into the sequence by a site-specific mutagenesis).

An identity of the modified product of the base sequence to the original base sequence is 80% or more, preferably 90% or more, further preferably 95% or more, further more preferably 97% or more.

The modified product of the base sequence, as long as having the functions of the enzyme or protein encoded in the original base sequence, may be a base sequence complementary to a base sequence well-known in the art, which hybridizes with the base sequence under a stringent condition, or a probe made from a part thereof.

The "stringent condition" refers to a condition where a specific hybridyzation occurs whereas a non-specific hybridyzation does not occur. For example, such condition is a condition where DNAs having a high sequence identity, e.g., a sequence identity of 80% or more, preferably 90% or more, more preferably 95% or more, further more preferably 97% or more, hybridize with each other, whereas DNAs having a low sequence identity, e.g., a sequence identity of less than 80%, do not hybridize with each other; or, a condition where hybridization may be maintained after one time, preferably 2 to 3 times of washing under a condition of 60°C, 1×SSC, 0.1% SDS, preferably a condition of 60°C, 0.1×SSC, 0.1% SDS, more preferably 68°C, 0.1×SSC, 0.1%, which is an ordinary condition for washing operation in Southern hybridization experiment.

A codon encoding the amino acids of the aforementioned enzyme or protein may vary depending on a usage frequency of the codon in a host for expression of the enzyme or protein, thereby varying the base sequence of the gene encoding the aforementioned enzyme or protein.

### [Method for transducing gene]

Examples of the method for transducing gen used in the method according to the example include methods well-known in the art.

Such methods include methods for transducing into a host cell a plasmid vector having a gene encoding a target protein such as enzyme and the like, via a method using microbial cells (competent cells) treated with calcium chloride, electroporation method, etc.; methods for transducing into a chromosome of a bacterium a phage vector with a gene encoding a target protein transduced therein by infecting microbial cells of the bacterium with the same; and the like.

It is preferable that in the gene transduction in this disclosure, a copy number of a target gene on a chromosome is increased to enhance an activity of the target protein. In the case of transducing a gene of an enzyme which is not originally possessed by a microbe as the host, it is preferable that the microbe contains 1 copy or more of the gene on its chromosome. Moreover, in the case of transducing a gene of an enzyme which is possessed by the microbe as the host, it is preferable that the microbe has a pluraility of copies, preferably 2 coplies or more, more preferably 3 copies or more of the gene on its chromosome.

In the gene transduction in this disclosure, the transduction of the target gene may also be achieved by transducing a plasmid carrying the gene on the host cell.

Here, increase of the copy number can also be accomplished by utilizing transposon or Mu phage to transfer the target gene onto the genome of the host.

Here, examples of the vector include a plasmid vector, a viral vector, etc., without being limited thereto. The vector may be either a DNA vector or an RNA vector. Examples of the vector include a monocistronic vector, a bicistronic vector, a polycistronic vector, etc.

The vector may be appropriately selected depending on the used host. Examples of the expression vector may include ColE-based plasmids typified by pBR322 derivatives, pACYC-based plasmids having a p15A origin, pSC-based plasmids, and mini F plasmids derived from an F factor of Bac and the like in Escherichia coli (E. coli).

Construction of the vector may be performed with a genetic engineering method such as PCR method, crossover PCR method, In-fusion method, *λ*-Red method, etc., by appropriately using an enzyme such as a restriction enzyme, a DNA polymerase, a ligase, etc.

Note that it is preferable that a system used for the aforementioned gene transduction is constructed with an ordinary method so as to be capable of inducing the expression of the target gene in the cultivation described below. Examples of such induction include IPTG induction, arabinose induction, etc. Here, examples of a promoter used in the vector include a tryptophan promoter such as trc, tac and the like, lac promoter, T7 promoter, T5 promoter, T3 promoter, SP6 promoter, arabinose-inducible promoter, cold-shock promoter, tetracycline-inducible promoter, etc.

Note that in this disclosure, it is preferable to transduce the isoprene synthase into a cytoplasm of Pantoea ananatis with the aforementioned vector, and to further perform chromosome fixation with enzymes involved in an upstream of the mevalonic acid pathway as artificial operons, and to perform chromosome fixation to mevalonate kinase independently with other enzymes as artificial operons among enzymes involved in a downstream of the mevalonic acid pathway, to thereby fix the same to a chromosome of Pantoea ananatis.

Details of transformant cultivation would be described in the following. [Cultivation method]

Examples of methods of culturing the transformant include batch cultivation, feeding cultivation (fed batch cultivation), continuous cultivation (perfusion cultivation), etc. In particular, from the viewpoint of the capability of industrial production of isoprene, feeding cultivation and continuous cultivation are preferable.

Batch cultivation is a method in which the cultivation is performed by preparing a new culture medium in each time of cultivation, and adding the transformant therein, without adding the culture medium, etc. In batch cultivation, the transformant follows a growth cycle including a lag phase, a logarithmic growth phase and a stationary phase, where the production volume of isoprene increases in the logarithmic growth phase and the stationary phase.

Feeding cultivation is a method in which the transformant is added into a culture medium to perform cultivation, and the culture medium and components of the culture medium are appropriately added during the cultivation. In feeding cultivation, it is possible to optimize a growth rate by adjusting a cell density, or to maintain a productivity of isoprene by diluting harmful substances accumulated in the culture medium.

The continuous cultivation method is a method in which a certain amount of the medium is continuously supplied to a culture tank at a constant rate while the same amount of the medium is removed. In continuous cultivation, a constant culture environment may be maintained. More specifically, according to this cultivation, it is possible to maintain the logarithmic growth phase mainly with respect to the transformant in the culture medium, and thus the productivity of isoprene may be easily maintained. Moreover, accumulation of metabolic byproducts and dead cells, which potentially have adverse effects on the growth of the transformant, can be prevented.

### [Culture condition]

A culture condition is not particularly limited as long as capable of expressing the protein of this disclosure, and a standard cell culture condition can be used.

The cultivation may be performed under an aerobic, oxygen-free, or anaerobic condition depending on a nature of the host cell.

The culture temperature is preferably 20 to 37°C, the culture atmosphere is preferably about 6% to about 84% of CO₂ concentration, and the pH value is preferably about 5 to about 9.

### [Culture medium]

The culture medium used in the method according to the example is either a natural medium or a synthesized medium, and is a culture medium containing at least a carbon source for forming an isoprene skeleton, and containing optionally a nitrogen source, an organic trace nutrient source, an inorganic matter (mineral), etc.

The carbon source may include carbohydrates such as monosaccharides, disaccharides, oligosaccharides, polysaccharides and invert sugars; compounds having one carbon atom (hereinafter referred to as a C1 compound); lipids (oils, etc.); fatty acids; glycerine fatty acid esters; polypeptides; renewable carbon sources; yeast extracts; phospholipids; glycerolipids; glycerol; acetate, etc.

Such carbon sources may be used alone or in a combination of two or more.

The carbon sources would be described in details in the following.

Examples of the monosaccharides among the carbohydrates may include triose such as ketotriose (dihydroxyacetone) and aldotriose (glyceraldehyde); tetrose such as ketotetrose (erythrulose) and aldotetrose (erythrose, threose); pentose such as ketopentose (ribulose, xylulose), aldopentose (ribose, arabinose, xylose, lyxose) and deoxysaccharide (deoxyribose); hexose such as ketohexose (psychose, fructose, sorbose, tagatose), aldohexose (allose, altrose, glucose, mannose, gulose, idose, galactose, tallose), and deoxysaccharide (fucose, fucrose, rhamnose); and heptose such as sedoheptulose. C6 sugars such as fructose, mannose, galactose and glucose; and C5 sugars such as xylose and arabinose are preferable.

Examples of the disaccharides may include sucrose, lactose, maltose, trehalose, turanose, and cellobiose. Sucrose and lactose are preferable.

Examples of the oligosaccharides may include trisaccharides such as raffinose, melezitose and maltotriose; tetrasaccharides such as acarbose and stachyose; and other oligosaccharides such as fructooligosaccharide (FOS), galactooligosaccharide (GOS) and mannan-oligosaccharide (MOS).

Examples of the polysaccharides may include glycogen, starch (amylose, amylopectin), cellulose, dextrin, and glucan (*β*1,3-glucan). Starch and cellulose are preferable.

Examples of the invert sugar include one obtained by hydrolyzing sucrose.

Examples of the compound having one carbon atom (C₁ compound) include methanol, formaldehyde, formate, carbon monoxide and carbon dioxide.

Examples of the lipid may include substances containing one or more saturated or unsaturated fatty acids of C₄ or more. In particular, an oil which is liquid at room temperature is preferable.

Examples of the oil may include plant oils such as soybean, corn, canola, Jatropha, palm, peanut, sunflower, coconut, mustard, cotton seed, palm kernel oil, olive, safflower, sesame, linseed, oily microbial cells, Chinese tallow tree, and animal oils.

Examples of the fatty acid may include compounds represented by a formula RCOOH ("R" represents a hydrocarbon group), which include unsaturated fatty acids and saturated fatty acids. Here, the unsaturated fatty acid is a compound having at least one double bond between two carbon atoms in "R", and examples of the unsaturated fatty acid may include oleic acid, vaccenic acid, linoleic acid, palmitelaidic acid and arachidonic acid. The saturated fatty acid is a compound where the "R" is a saturated aliphatic group, and examples of the saturated fatty acid may include docosanoic acid, eicosanoic acid, octadecanoic acid, hexadecanoic acid, tetradecanoic acid, and dodecanoic acid. C₂ to C₂₂ fatty acids are preferable as the fatty acid, and C₁₂ fatty acid, C₁₄ fatty acid, C₁₆ fatty acid, C₁₈ fatty acid, C₂₀ fatty acid and C₂₂ fatty acid are more preferable.

The carbon source may also include salts and derivatives of these fatty acids and salts of these derivatives. Examples of the salt may include lithium salts, potassium salts and sodium salts.

Examples of the glycerine fatty acid ester include monoglyceride, diglyceride and triglyceride.

Examples of the polypeptide include microbial polypeptide and plant polypeptide. Here, examples of the microbial protein may include polypeptides obtainable from a yeast or bacterium, and examples of the plant protein may include polypeptides obtainable from soybean, corn, canola, Jatropha, palm, peanut, sunflower, coconut, mustard, cotton seed, palm kernel oil, olive, safflower, sesame and linseed.

Examples of the renewable carbon source may include biomass carbon sources. In particular, entirely or partially hydrolyzed biomass carbon sources are preferable. Examples of the biomass carbon source may include cellulose-based substrates such as waste materials of woods, papers and pulps, leafy plants, and fruit pulps; and partial plants such as stalks, grain particles, roots and tubers. Examples of the plants to be used as the biomass carbon source may include corn, wheat, rye, sorghum, triticale, rice, millet, barley, cassava, legumes such as peas, potato, sweet potato, banana, sugar cane, and tapioca. When the renewable carbon source such as biomass is added to the culture medium, the carbon source is preferably pretreated. Examples of the pretreatment may include an enzymatic pretreatment, a chemical pretreatment, and a combination thereof.

Examples of the carbon source may also include combinations of carbohydrate such as glucose with the lipid(s), the oil(s), the fats, the fatty acid(s) and glycerin fatty acid(s) ester(s).

Examples of the carbon source may also include the yeast extract and a combination of the yeast extract with the other carbon source such as glucose. The combination of the yeast extract with the C1 compound such as carbon dioxide and methanol is preferable.

For a nitrogen source, inorganic ammonium salts such as ammonium sulfate, ammonium chloride and ammonium phosphate, organic nitrogen such as hydrolyzed soybeans, ammonia gas, ammonia water, and the like can be used.

It is desirable to include required substances such as vitamin B1 and L-homoserine, or yeast extract and the like in an appropriate amount as an organic trace nutrient source.

For an inorganic matter (mineral), in addition thereto, potassium phosphate, magnesium sulfate, iron ion, manganese ion, and the like may be added in small amounts if necessary.

The culture medium used in the method according to the example is preferably a commercially available standard culture medium.

The standard culture medium is not particularly limited, and examples of the culture medium may include ready-made general media such as Luria Bertani (LB) broth, Sabouraud dextrose (SD) broth, and yeast medium (YM) broth. The medium suitable for the cultivation of the specific host can be selected appropriately for the use.

The isoprene produced with the cultured transformant is emitted as a gas from a fermentation tank. The isoprene gas is taken into a gas collecting apparatus, liquefied by cooling with a vapor trap, etc. equipped with a cooling apparatus, and thus can be recovered as liquid isoprene. At this time, the isoprene gas mainly passes through an adsorbent for adsorbing isoprene (e.g., hydrophobic silica gel and the like), and then its pressure is reduced as compared to the time of adsorption. Thereby, the gas with isoprene as a main component can be recovered (WO2014/156997A1). Moreover, it is preferable that the isoprene gas is subjected to condensation removal of moisture via cooling, in order to have moisture removed.

Examples of the isoprene recovery apparatus include the apparatus as described in the example described above.

The fermentation method is as described in the above.

### --Polymerization catalyst composition-

An example of a polymerization catalyst composition used in the method for producing polyisoprene of the examples of this disclosure would be described in the following.

This polymerization catalyst composition necessarily contains:
- a rare earth element compound (hereinafter referred to as "component (A)" as well); and
- an organometallic compound (hereinafter referred to as "component (B)" as well).

Here, this polymerization catalyst composition may further contain:
- an aluminoxane compound (hereinafter referred to as "component (C)" as well);
- a halogen compound (hereinafter referred to as "component (D)" as well);
- an ionic compound (hereinafter referred to as "component (E)" as well); and
- a compound capable of serving as an anionic ligand (hereinafter referred to as "component (F)" as well).

### --Rare earth element compound (component (A))--

The component (A) may be a rare earth element-containing compound or a reaction product of the rare earth element-containing compound and a Lewis base, which has metal-nitrogen bond (M-N bond).

Note that examples of the rare earth containing compound include to a compound containing scandium, yttrium, or lanthanoid composed of elements with atomic numbers from 57 to 71. Specific examples of the lanthanoid element include lanthanum, cerium, praseodymium, neodymium, promethium, samarium, europium, gadolinium, terbium, dysprosium, holmium, erbium, thulium, ytterbium, and lutetium.

Examples of the Lewis base include tetrahydrofuran, diethyl ether, dimethylaniline, trimethylphosphine, lithium chloride, neutral olefins, and neutral diolefins.

Here, it is preferable that the rare earth element-containing compound or the reaction product of the rare earth element-containing compound and the Lewis base has no bond between the rare earth element and carbon. When the reaction product of the rare earth element-containing compound and the Lewis base has no bond between the rare earth element and carbon, an obtained compound is stable and easy to handle.

Note that the component (A) may be used alone or in a combination of two or more.

Here, the component (A) is preferably a compound represented with the formula (S1):

M-(NQ¹)(NQ²)(NQ³) ... (S1)

where M is at least one element selected from scandium, yttrium or lanthanide elements; NQ¹, NQ², and NQ³ are amide groups and may be either the same or different from one another; and the compound has three M-N bonds.

According to the aforementioned configuration, it is possible to use a compound having 3 M-N bonds as the component (A), in which each bond is chemically equivalent, rendering the structure of the compound stable and easy to handle. Moreover, according to the aforementioned configuration, the catalytic activity in the reaction system may be further improved. Therefore, the reaction time may be further shortened, and the reaction temperature may be further raised.

The amide groups represented by NQ¹, NQ² and NQ³ may be any one of an aliphatic amide group such as a dimethylamide group, a diethylamide group, and a diisopropyl amide group; a phenylamide group, a 2,6-di-tert-butylphenyl amide group, a 2,6-diisopropylphenyl amide group, a 2,6-dineopentilphenyl amide group, a 2-tert-butyl-6-isopropyphenyl amide group, a 2-tert-butyl-6-neopentilphenyl amide group, and a 2-isopropyl-6-neopentilphenyl amide group; an arylamide group such as a 2,4,6-tert-butylphenyl amide group; and a bis-trialkyl silylamide group such as a bis-trimethyl silylamide group. Among them, the bis-trimethylsilyl amide group is preferable.

Such amide groups may be used alone or in a combination of two or more.

### --Organometallic compound (component (B))--

The component (B) is a compound represented with the formula (S2):

YR⁴ₐR⁵_{b}R⁶_{c}... (S2)

where Y is a metallic element selected from the group consisting of Group 1, Group 2, Group 12 and Group 13; R⁴ and R⁵ are C1 to C10 hydrocarbon group or hydrogen atom, and may be either identical or different; R⁶ is a C1 to C10 hydrocarbon group; R⁶ may be either identical to or different from the R⁴ or R⁵; if Y is a Group 1 metallic element, a is 1, and b, c are 0; if Y is a Group 2 metallic element or a Group 12 metallic element, a and b are 1, and c is 0; and if Y is a Group 13 metallic element, a, b and c are 1.

The component (B) is preferably an organoaluminum compound represented with the formula (S3):

AlR⁷R⁸R⁹ ... (S3)

where R⁷ and R⁸ are C1 to C10 hydrocarbon group or hydrogen atom, and may be either identical or different; R⁹ is a C1 to C10 hydrocarbon group; and R⁹ may be either identical to or different from the R⁷ or R⁸.

As the organoaluminum compound in general formula (X), trimethyl aluminum, triethyl aluminum, tri-n-propyl aluminum, triisopropyl aluminum, tri-n-butyl aluminum, triisobutyl aluminum, tri-t-butyl aluminum, tripentyl aluminum, trihexyl aluminum, tricyclohexyl aluminum, trioctyl aluminum; diethylaluminum hydride, di-n-propyl aluminum hydride, di-n-butyl aluminum hydride, diisobutyl aluminum hydride, dihexyl aluminum hydride, diisohexyl aluminum hydride, dioctyl aluminum hydride, diisooctyl aluminum hydride; ethyl aluminum dihydride, n-propyl aluminum dihydride, isobutyl aluminum dihydride may be used, among which triethyl aluminum, triisobutyl aluminum, diethylaluminum hydride, diisobutyl aluminum hydride are preferable.

Such organoaluminum compounds may be used alone or in a combination of two or more.

### --Aluminoxane compound (compound (C))--

The compound (C) is a compound obtainable by bringing an organoaluminum compound into contact with a condensing agent.

By using the component (C), the catalytic activity in the polymerization reaction system may be further improved. Therefore, the reaction time may be further shortened, and the reaction temperature may be further raised.

Here, examples of the organoaluminum compound include trialkyl aluminum such as trimethyl aluminum, triethyl aluminum, triisobutyl aluminum, etc., and a mixture thereof. Among them, a mixture of trimethyl aluminum and tributyl aluminum is preferable.

Examples of the condensing agent include one ordinarily used in the art.

Examples of the component (C) include an aluminoxane represented with the formula (S4):

-(Al(R¹⁰)O)ₙ- ... (S4)

where R¹⁰ represents a C1 to C10 hydrocarbon group, and may be one that is partially substituted for by a halogen atom and/or an alkoxy group; R¹⁰ may be either identical or different among repeating units; and n is 5 or more.

The molecular structure of the aforementioned aluminoxane may be either straight chain or cyclic. Moreover, n is preferably 10 or more. Further, examples of the hydrocarbon group of R¹⁰ include methyl group, ethyl group, propyl group, isobutyl group, etc. In particular, methyl group is preferable. Such hydrocarbon groups may be used alone or in a combination of two or more. The hydrocarbon group of R¹⁰ is preferably a combination of methyl group and isobutyl group.

Examples of the component (C) include TMAO, e.g., trade name: TMAO-341, manufactured by Tosoh Finechem Corporation.

Moreover, examples of the component (C) include MMAO, e.g., trade name: MMAO-3A, manufactured by Tosoh Finechem Corporation.

Further, examples of the component (C) include PMAO, e.g., trade name: TMAO-211, manufactured by Tosoh Finechem Corporation.

Among these, from the viewpoint of improving the effect of catalytic activity enhancement, TMAO and MMAO are preferable, and from the viewpoint of further improving the effect of catalytic activity enhancement, TMAO is more preferable.

### --Halogen compound (component (D))--

The component (D) is at least one compound selected from the group consisting of: a halogen containing compound, which is a Lewis acid (hereinafter referred to as "component (D-1)" as well), a complex compound of metal halides and a Lewis base (hereinafter referred to as "component (D-2)" as well), and an organic compound containing an active halogen (hereinafter referred to as "component (D-3)" as well).

These compounds react with the component (A), i.e., a rare earth element-containing compound or a reaction product of the rare earth element-containing compound and a Lewis base, which has M-N bond, thereby generating a cationic transition metal compound, a halogenated transition metal compound, and/or a transition metal compound in a state where a transition metal center has insufficient electrons.

By using the component (D), the cis-1,4-bond content of polyisoprene may be raised.

Examples of the component (D-1) include a halogen containing compound containing an element of Group 3, Group 4, Group 5, Group 6, Group 8, Group 13, Group 14 or Group 15. In particular, an aluminum halide or an organometallic halide is preferable.

Examples of the halogen containing compound, which is a Lewis acid, include titanium tetrachloride, tungsten hexachloride, tri(pentafluorophenyl) borate, methyl aluminum dibromide, methyl aluminum dichloride, ethyl aluminum dibromide, ethyl aluminum dichloride, butyl aluminum dibromide, butyl aluminum dichloride, dimethyl aluminum bromide, dimethyl aluminum chloride, diethyl aluminum bromide, diethyl aluminum chloride, dibutyl aluminum bromide, dibutyl aluminum chloride, methyl aluminum sesquibromide, methyl aluminum sesquichloride, ethyl aluminum sesquibromide, ethyl aluminum sesquichloride, aluminum tribromide, tri(pentafluorophenyl aluminum, dibutyltin dichloride, tin tetrachloride, phosphorus trichloride, phosphorus pentachloride, antimony trichloride and antimony pentachloride. In particular, ethyl aluminum dichloride, ethyl aluminum dibromide, diethyl aluminum chloride, diethyl aluminum bromide, ethyl aluminum sesquichloride and ethyl aluminum sesquibromide are preferable.

As the halogen, chlorine or bromine is preferable.

The halogen containing compound, which is a Lewis acid, may be used alone or in a combination of two or more.

Examples of the metal halide used in the component (D-2) include beryllium chloride, beryllium bromide, beryllium iodide, magnesium chloride, magnesium bromide, magnesium iodide, calcium chloride, calcium bromide, calcium iodide, barium chloride, barium bromide, barium iodide, zinc chloride, zinc bromide, zinc iodide, cadmium chloride, cadmium bromide, cadmium iodide, mercury chloride, mercury bromide, mercury iodide, manganese chloride, manganese bromide, manganese iodide, rhenium chloride, rhenium bromide, rhenium iodide, copper chloride, copper iodide, silver chloride, silver bromide, silver iodide, gold chloride, gold iodide, gold bromide, etc. In particular, magnesium chloride, calcium chloride, barium chloride, zinc chloride, manganese chloride and copper chloride are preferable, and magnesium chloride, zinc chloride, manganese chloride and copper chloride are more preferable.

The Lewis base used in the component (D-2) is preferably a phosphorus compound, a carbonyl compound, a nitrogen compound, an ether compound, or an alcohol.

For example, tributyl phosphate, tri-2-ethylhexyl phosphate, triphenyl phosphate, tricresyl phosphate, triethylphosphine, tributylphosphine, triphenylphosphine, diethylphosphino ethane, diphenylphosphino ethane, acetylacetone, benzoylacetone, propionitrileacetone, valerylacetone, ethyl acetylacetone, methyl acetoacetate, ethyl acetoacetate, phenyl acetoacetate, dimethyl malonate, diethyl malonate, diphenyl malonate, acetic acid, octanoic acid, 2-ethylhexanoic acid, oleic acid, stearic acid, benzoic acid, naphthenic acid, versatic acid (a trade name manufactured by Shell Chemicals, which is a synthetic acid containing a mixture of isomers of C₁₀ monocarboxylic acid), triethylamine, N,N-dimethylacetamide, tetrahydrofuran, diphenyl ether, 2-ethylhexyl alcohol, oleyl alcohol, stearyl alcohol, phenol, benzyl alcohol, 1-decanol, lauryl alcohol and the like may be mentioned. In particular, tri-2-ethylhexyl phosphate, tricresyl phosphate, acetylacetone, 2-ethylhexane acid, versatic acid, 2-ethylhexyl alcohol, 1-decanol, and lauryl alcohol are preferable.

The Lewis base mentioned above is brought to reaction at a ratio of 0.01 to 30 mol, preferably 0.5 to 10 mol per mole of the metal halide mentioned above. A reaction product with the Lewis base at this ratio allows a reduction in metal remaining in the polymer.

Examples of the component (D-3) include benzyl chloride and the like.

### --Ionic compound (component (E))--

As the component (E) as described above may be an ionic compound and the like that includes non-coordinating anion and cation.

Examples of the non-coordinating anion include tetravalent boron anion, such as tetraphenyl borate, tetrakis(monofluorophenyl)borate, tetrakis(difluorophenyl)borate, tetrakis(trifluorophenyl)borate, tetrakis(tetrafluorophenyl)borate, tetrakis(pentafluorophenyl)borate, tetrakis(tetrafluoromethylphenyl)borate, tetra(tolyl) borate, tetra(xylyl)borate, (triphenyl,pentafluorophenyl)borate, [tris(pentafluorophenyl)phenyl]borate, tridecahydride-7,8-dicarbaundecaborate, etc.

Such non-coordinating anion may be used alone or in a combination of two or more.

Examples of the cation include carbonium cation, oxonium cation, ammonium cation, phosphonium cation, cycloheptatrienyl cation, and ferrocenium cation having transition metal. Here, examples of the carbonium cation include trisubstituted carbonium cation such as triphenyl carbonium cation, tri(substituted phenyl) carbonium cation and the like. Moreover, examples of the tri(substituted phenyl) carbonium cation include tri(methylphenyl) carbonium cation, tri(dimethylphenyl) carbonium cation and the like.

Examples of the ammonium cation include trialkyl ammonium cation such as trimethyl ammonium cation, triethyl ammonium cation, tripropyl ammonium cation, and tributyl ammonium cation; N,N-dialkyl anilinium cation such as N,N-dimethyl anilinium cation, N,N-diethyl anilinium cation, N,N-2,4,6-pentamethyl anilinium cation and the like; and dialkyl ammonium cation such as diisopropyl ammonium cation, dicyclohexyl ammonium cation and the like.

Examples of the phosphonium cation include triarylphosphonium cation such as triphenylphosphonium cation, tri(methylphenyl)phosphonium cation, tri(dimethylphenyl)phosphonium cation and the like.

Such cation may be used alone or in a combination of two or more.

In particular, preferred examples include N,N-dimethylanilinium tetrakis(pentafluorophenyl)borate and triphenylcarbonium tetrakis(pentafluorophenyl)borate.

### --Compound capable of serving as an anionic ligand--

Examples of the component (F) include an anionic tridentate ligand precursor represented with the formula (S5)(see Organometallics, 23, p 47784787 (2004)). 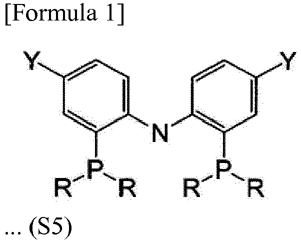 (in the formula, R represents an alkyl group or an aryl group, Y represents at least one selected from the group consisting of a hydrogen, an alkyl group, a halogen group, and a silyl group)

In particular, a PNP ligand such as bis(2-diphenylphosphinophenyl) amine and the like may be mentioned.

According to the method for producing polyisoprene of this disclosure, it is possible to obtain the effect of the isoprene-containing composition of this disclosure, i.e., the capability of producing a polyisoprene with a comparatively high molecular weight.

A weight ratio of each component in the polymerization catalyst composition used in the method for producing polyisoprene of the examples of this disclosure would be described in the following.

From the viewpoint of improving the catalytic activity in the reaction, a molar ratio of the component (B) (the organometallic compound) to the component (A) is preferably 1 to 50.

From the viewpoint of improving the catalytic activity in the reaction, a molar ratio of the aluminum in the component (C) to the rare earth element in the component (A) is preferably 10 to 1000.

From the viewpoint of improving the catalytic activity in the reaction, a molar ratio of the component (D) (the halogen compound) to the component (A) is preferably 0.1 to 50.

From the viewpoint of improving the catalytic activity in the reaction, a molar ratio of the component (E) (the ionic compound) to the component (A) is preferably 0.1 to 10.

Here, in the method for producing polyisoprene of the examples of this disclosure, from the viewpoint of sufficiently activating the polymerization reaction, a compounding amount of the rare earth element compound is preferably 0.018 parts by mass or more, more preferably 0.02 parts by mass or more, preferably 0.55 parts by mass or more, more preferably 0.6 parts by mass or more, further preferably 0.65 parts by mass or more, particularly preferably 0.7 parts by mass or more per 100 parts by mass of the isoprene-containing composition. Moreover, from the viewpoint of reducing the risk of deactivating the polymerization reaction in contrary, the compounding amount is preferably 0.95 parts by mass or less, more preferably 0.9 parts by mass or less, further preferably 0.85 parts by mass or less, particularly preferably 0.8 parts by mass or less per 100 parts by mass of isoprene-containing composition.

Here, in the method for producing polyisoprene of the examples of this disclosure, from the viewpoint of sufficiently activating the polymerization reaction, a compounding amount of the organometallic compound is necessarily 0.6 parts by mass or more, preferably 0.9 parts by mass or more, more preferably 1.1 parts by mass or more per 100 parts by mass of the isoprene-containing composition.

Moreover, from the viewpoint of reducing the risk of deactivating the polymerization reaction in contrary, the compounding amount of the organometallic compound is necessarily 3.0 parts by mass or less, preferably 1.6 parts by mass or less, more preferably 1.5 parts by mass or less or less per 100 parts by mass of the isoprene-containing composition.

### (Polyisoprene)

The polyisoprene (hereinafter referred to as "the polyisoprene" as well) produced with the method for producing polyisoprene of the examples of this disclosure would be described in the following.

The number-average molecular weight (Mn) of the polyisoprene is preferably 1,000,000 or more, more preferably 1,300,000 or more.

The molecular weight distribution (Mw/Mn) thereof is preferably 2.5 or less, more preferably 2.0 or less.

Note that the number-average molecular weight (Mn) and the molecular weight distribution (Mw/Mn) may be measured with the method as described in the examples mentioned below.

The cis-1,4-bond content of the polyisoprene is preferably 98% or more, more preferably 99% or more, without being limited thereto. A high value may enhance an elongation-induced crystallization ability of the polyisoprene, and enhance an elasticity of the polyisoprene.

A trans-1,4-bond content of the polyisoprene is preferably 2.0% or less, more preferably 1.0% or less, without being limited thereto. A lower value may enhance the elongation-induced crystallization ability of the polyisoprene, and enhance the elasticity of the polyisoprene.

A 1,2-vinyl bond content of the polyisoprene is preferably 2.0% or less, more preferably 1.0% or less, without being limited thereto. A lower value may enhance the elongation-induced crystallization ability of the polyisoprene, and enhance the elasticity of the polyisoprene.

A 3,4-vinyl bond content of the polyisoprene is preferably 2.0% or less, more preferably 1.0% or less, without being limited thereto. A lower value may enhance the elongation-induced crystallization ability of the polyisoprene, and enhance the elasticity of the polyisoprene.

### (Rubber composition)

The rubber composition containing the polyisoprene (hereinafter referred to as "the rubber composition" as well) would be described in the following. Here, the polyisoprene of this disclosure may be a rubber component in the rubber composition.

The rubber composition may contain rubber components other than the polyisoprene, and may further contain a filler, an age resistor, a softener, a stearic acid, a zinc oxide, a vulcanization accelerator, a vulcanizing agent, an oil, a sulfur, etc.

The rubber composition may be produced with a method well-known to a skilled person.

### (Tire, rubber product)

A tire may be produced by using the rubber composition. Here, any member of the tire may be produced by using the rubber composition. The tire may be produced with a method well-known to a skilled person.

Moreover, rubber products other than tire, such as footwear, belt, flooring, etc., may be produced by using the rubber composition as well.

### EXAMPLES

The present disclosure is described in more detail below with reference to Examples, by which the present disclosure is not intended to be limited in any way.

Note that the following examples use Pantoea ananatis as the host cell, one derived from Mucuna as the isoprene synthase, one derived from Saccharomyces cerevisiae, Enterococcus faecalis or Metanocella pardicola as the enzyme involved in the mevalonic acid pathway. Here, the isoprene synthase is transduced into the host cell via chromosome fixation of the enzyme involved in the mevalonic acid pathway by using a expression plasmid.

### (Example 1)

### (1) Preparation of isoprene-containing composition (preparation of fermented isoprene)

### (1-1) Preparation of Plasmid for Upstream of MVA Pathway for Chromosome Fixation

### (1-1-1) Construction of Arabinose-Inducible Plasmid for Expressing Mevalonate Pathway Upstream Gene (mvaES [Enterococcus faecalis (E. faecalis)]) Derived from E. faecalis, pMW-Pₐᵣₐ-mvaES-Tₜᵣₚ

### (1-1-1-1) Chemical Synthesis and Cloning of mvaES (E. faecalis) Gene

A nucleotide sequence (GenBank/EMBL/DDBJ accession ID AF290092.1) and an amino acid sequence (mvaS, GenPept accession ID AAG02438.1, mvaE, GenPept accession ID AAG02439.1) of the mvaES gene encoding the mevalonate pathway upstream gene (mvaES) (Enterococcus faecalis) (E. faecalis) derived from E. faecalis are known publicly (see Wilding, E I et al., J. Bacteriol. 182 (15), 4319-4327 (2000), which is incorporated herein by reference in its entirety). Based on this information, a mvaE gene and a mvaS gene in which their codon usages had been optimized for E. coli were designed and designated as EFmvaE and EF mvaS, respectively. Nucleotide sequences of EFmvaE and EFmvaS are shown in SEQ ID NO:1 and SEQ ID NO:2, respectively. DNA sequences of EFmvaE and EFmvaS prepared by chemical synthesis were cloned into a plasmid for expression pUC57 (supplied from GenScript), and designated as pUC57-EFmvaE and pUC57-EFmvaS, respectively. Nucleotide sequences of pUC57-EFmvaE and pUC57-EFmvaS are shown in SEQ ID NO:3 and SEQ ID NO:4, respectively.

### (1-1-1-2) Preparation for construction of Plasmid pMW-P_{trc}-mvaES-Tₜᵣₚ Used for In-Fusion Method

A plasmid pMW-P_{trc}-mvaES-Tₜᵣₚ used for the In-fusion method was constructed according to the following procedure.

A plasmid pMW219 (supplied from Nippon Gene, Part number: 310-02571) was digested with SmaI, and this digested plasmid was purified. The resulting plasmid was designated as pMW219/SmaI.

In order to obtain a gene of a trc promoter (P_{trc}) region, PCR was carried out using a plasmid pTrcHis2B having the P_{trc} region as the template and using synthesized oligonucleotides consisting of the nucleotide sequences of SEQ ID NO:5 and SEQ ID NO:6 as primers.

In order to obtain a mvaE gene portion, PCR was carried out using the plasmid pUC57-EFmvaE as the template and using synthesized oligonucleotides consisting of the nucleotide sequences of SEQ ID NO:7 and SEQ ID NO:8 as the primers.

In order to obtain a mvaS gene portion, PCR was carried out using the plasmid pUC57-EFmvaS as the template and using synthesized oligonucleotides consisting of the nucleotide sequences of SEQ ID NO:8 and SEQ ID NO:9 as the primers.

In order to obtain a gene of a trp terminator (Tₜᵣₚ) region, PCR was carried out using a plasmid pSTV-P_{tac}-Tₜᵣₚ having the Tₜᵣₚ region as the template and using synthesized oligonucleotides consisting of the nucleotide sequences of SEQ ID NO:10 and SEQ ID NO:11 as the primers.

In the above four PCR cases, PrimeStar polymerase (supplied from TaKaRa Bio) was used as the enzyme, a reaction solution was prepared according to instructions provided by the supplier of the enzyme, and the reaction of a cycle of 98°C for 10 seconds, 55°C for 5 seconds and 72°C for 60 seconds/kb was repeated 30 times. As a result, the PCR product comprising the gene of the P_{trc} region, the mvaE gene, the mvaS gene and the gene of the Tₜᵣₚ region was obtained.

### (1-1-1-3) Construction of Plasmid pMW-P_{trc}-mvaES-Tₜᵣₚ Used for In-Fusion Method

Subsequently, PCR was carried out using the purified PCR product comprising P_{trc} and the purified PCR product comprising the mvaE gene as the template and using synthesized oligonucleotides consisting of SEQ ID NO:5 and SEQ ID NO:8 as the primers. Also PCR was carried out using the purified PCR product comprising the mvaS gene and the purified PCR product comprising Tₜᵣₚ as the template and using synthesized oligonucleotides consisting of SEQ ID NO:9 and SEQ ID NO:12 as the primers.

As a result, the PCR product comprising the gene of the P_{trc} region and the mvaE gene, and the PCR product comprising the mvaS gene and the gene of the Tₜᵣₚ region were obtained.

Subsequently, the PCR product comprising the gene of the P_{trc} region and the mvaE gene, the PCR product comprising the mvaS gene and the gene of the Tₜᵣₚ region, and the plasmid pMW219/SmaI digested above were ligated using In-Fusion HD Cloning Kit (supplied from Clontech). The resulting plasmid was designated as pMW-P_{trc}-mvaES-Tₜᵣₚ. A sequence of obtained pMW-P_{trc}-mvaES-Tₜᵣₚ, is shown in SEQ ID NO:13.

### (1-1-1-4) Construction of Plasmid pMW-Pₐᵣₐ-mvaES-Tₜᵣₚ

The arabinose-inducible plasmid for expression of a mevalonate pathway upstream gene, pMW-Pₐᵣₐ-mvaES-Tₜᵣₚ was constructed according to the following procedure.

PCR was carried out using the plasmid pMW-P_{trc}-mvaES-Tₜᵣₚ prepared in (1-1-3) as the template and using synthesized oligonucleotides consisting of the nucleotide sequences of SEQ ID NO:14 and SEQ ID NO:15 as the primers.

PCR was carried out using a plasmid pKD46 (see Proc. Natl. Acad. Sci. USA, 2000, vol. 97, No. 12, p 6640-6645, which is incorporated herein by reference in its entirety) comprising a gene of a P_{araC} region, an araC gene and a gene of a P_{araBAD} region (hereinafter also collectively referred to as a "gene of a Pₐᵣₐ region") as the template and using synthesized oligonucleotides consisting of the nucleotide sequences of SEQ ID NO: 16 and SEQ ID NO:17 as the primers.

As a result, the PCR product comprising the plasmid pMW and the mvaES gene and the PCR product comprising the gene of the Pₐᵣₐ region were obtained. Purified these PCR products were ligated using In-Fusion HD Cloning Kit (supplied from Clontech). The resulting arabinose-inducible plasmid for expression of the mevalonate pathway upstream gene derived from E. faecalis (mvaES (E. faecalis)) was designated as pMW-Pₐᵣₐ-mvaES-Tₜᵣₚ. A nucleotide sequence of pMW-Pₐᵣₐ-mvaES-Tₜᵣₚ is shown in SEQ ID NO:18.

### (1-1-2) Construction of Integrative Conditional Replication Plasmid Possessing Mevalonate Pathway Upstream

A vector pAH162-*λ*attL-Tc^{R}-*λ*attR (Minaeva N I et al., BMC Biotechnol. 2008; 8:63, which is incorporated herein by reference in its entirety) was used in order to construct an integrative plasmid possessing an upstream gene and a downstream gene of the mevalonate pathway.

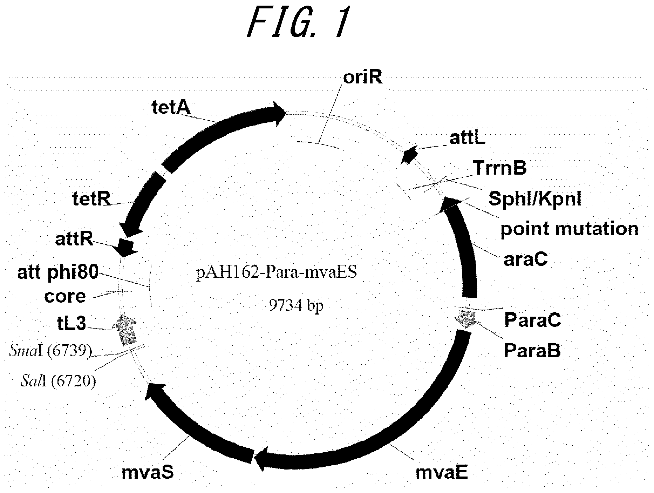

A KpnI-SalI fragment of pMW-Pₐᵣₐ-mvaES-Tₜᵣₚ was cloned into a recognition site for SphI-SalI in pAH162-*λ*attL-Tc^{R}-*λ*attR. As a result, a plasmid pAH162-Pₐᵣₐ-mvaES possessing an operon mvaES derived from E. faecalis under the control of the Para promoter and a repressor gene araC from E. coli was constructed (FIG. 1).

### (1-2) Preparation of Plasmid for Downstream of MVA Pathway for Chromosome Fixation

### (1-2-1) Construction of integrative plasmid pAH162-Km-Ptac-KDyI

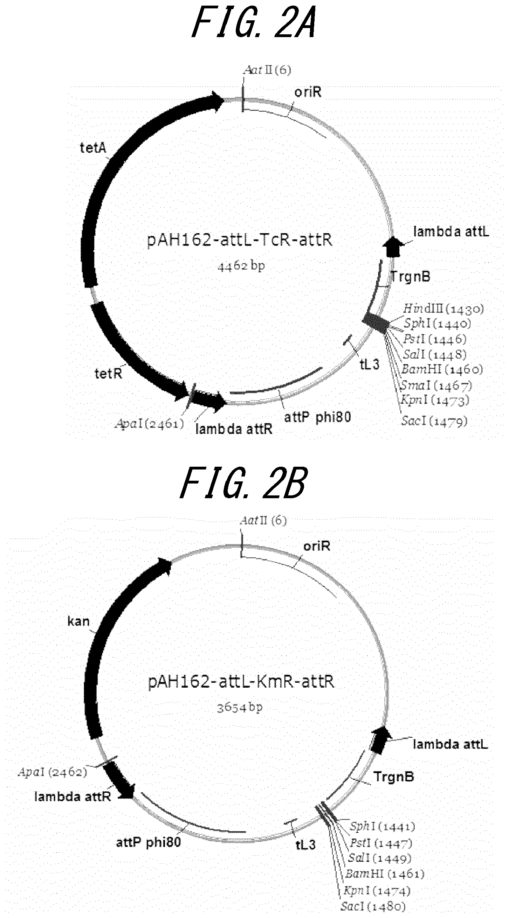

An AatII-ApaI fragment of an integrative plasmid pAH162-*λ*attL-Tc^{R}-*λ*attR (Minaeva N I et al. BMC Biotechnol. 2008; 8:63, which is incorporated herein by reference in its entirety) comprising a tetAR gene (FIG. 2A) was substituted with a DNA fragment obtained by PCR using synthesized oligonucleotides consisting of the nucleotide sequences of SEQ ID NO:19 (primer 11) and SEQ ID NO:20 (primer 12) as the primers and using a plasmid pUC4K (Taylor L A and Rose R E. Nucleic Acids Res. 16, 358, 1988, which is incorporated herein by reference in its entirety) as the template. As a result, pAH162-*λ*attL-Tm^{R}-*λ*attR was obtained (FIG. 2B).

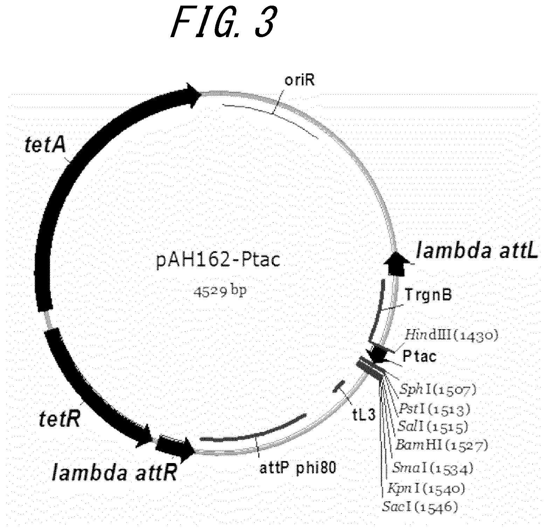

The Ptac promoter was inserted into a site HindIII-SphI in the integrative vector pAH162-*λ*attL-Tc^{R}-*λ*attR. As a result, an integrative vector pAH162-P_{tac} was constructed. The cloned promoter fragment was sequenced. A map of pAH162-P_{tac} is shown in FIG. 3.

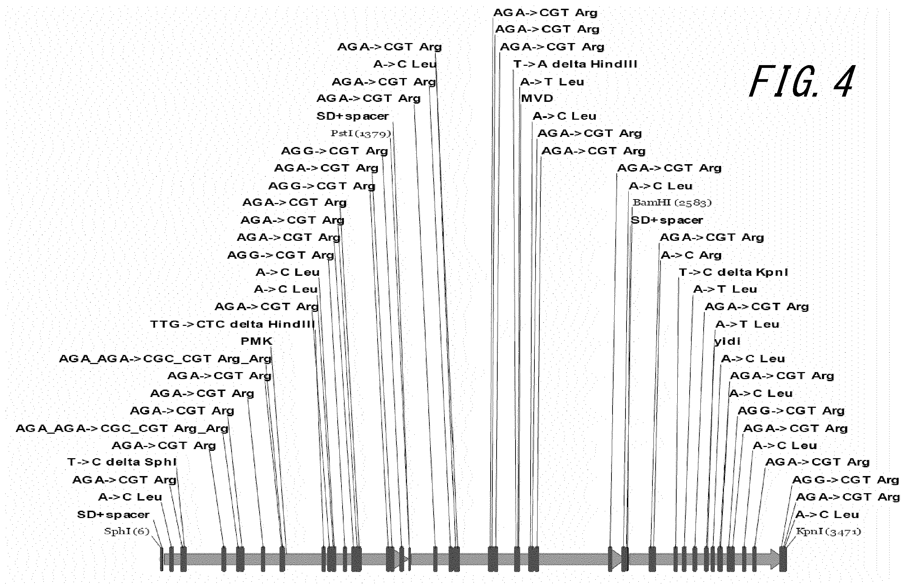

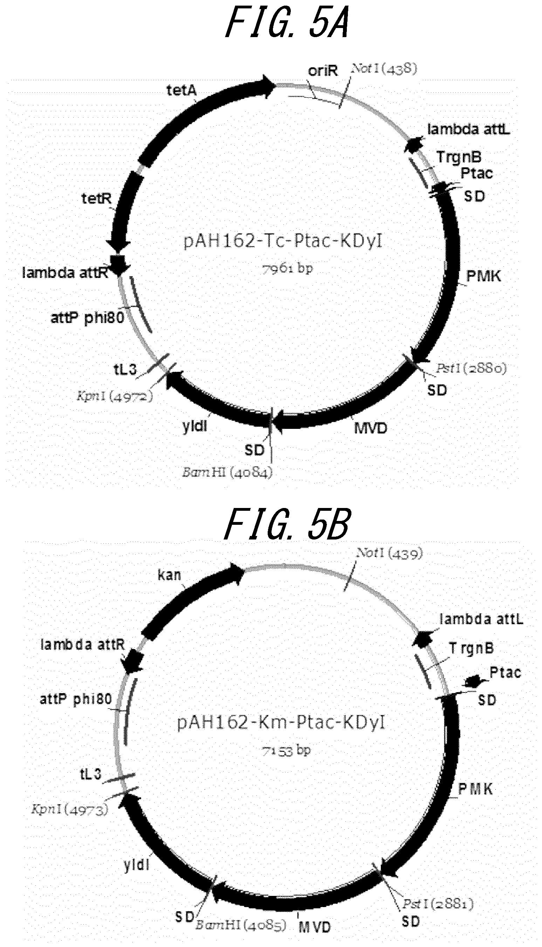

Regarding a DNA fragment holding the PMK gene, the MVD gene and the yIDI gene derived from S.cerevisiae (KDyloperon), a codon was optimized by substituting rare codons, and the chemically synthesized DNA fragment with the codon optimized was obtained from ATG Service Gene (Russia) (SEQ ID NO:21) (FIG. 4), and was subcloned into a site SphI-KpnI in the integrative vector pAH162-P_{tac}. The resulting plasmid pAH162-Tc-P_{tac}-KDyI holding an expression cassette P_{tac}-KDyI is shown in FIG. 5A. Subsequently, a NotI-KpnI fragment of pAH162-Tc-P_{tac}-KDyI holding a tetAR gene was substituted with a corresponding fragment of pAH162-*λ*attL-Tm^{R}-*λ*attR As a result, a plasmid pAH162-Km-P_{tac}-KDyI having a kanamycin resistant gene kan as a marker was obtained (FIG. 5B).

### (1-2-2) Construction of pAH162-P_{tac}-mvk (M.paludicola) intergrative plasmid

A chemically synthesized DNA fragment (SEQ ID NO:22) comprising a coding portion of a presumed mevalonate kinase gene (mvk gene) derived from SANAE (for complete genome sequence, see GenBank Accession Number AP011532) that was Methanocella paludicola and ligated to a standard SD sequence was cloned into a site PstI-KpnI in the above integrative expression vector pAH162-Ptac, to thereby obtain a pAH162-P_{tac}-mvk (M.paludicola) plasmid.

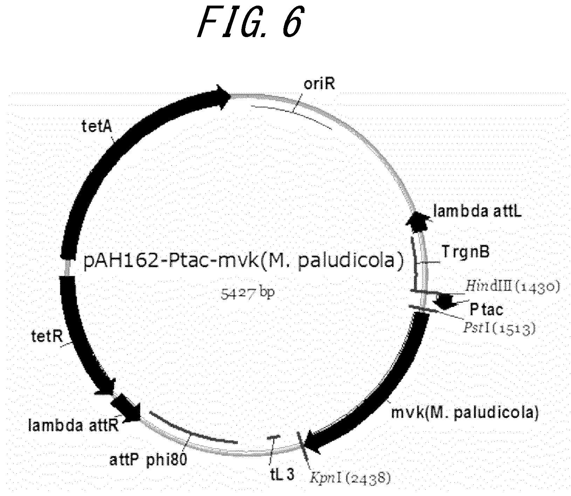

A map of the integrative plasmid pAH162-P_{tac}-mvk holding the mvk gene is shown in FIG. 6.

### (1-3) Construction of isoprene-producing bacterium

### (1-3-1) Construction of Recipient Strain SC17(0) ΔampC::attBₚₕᵢ₈₀ ΔampH::attBₚₕᵢ₈₀ Δcrt::P_{tac}-mvk (M.paludicola)

Chromosomal modifications ΔampH::attBₚₕᵢ₈₀ and ΔampC::attBₚₕᵢ₈₀ were introduced into P. ananatis SC17(0) stepwise using two step technique comprising λRed dependent integration of a PCR-amplified DNA fragment comprising the gene kan flanking to attLₚₕᵢ₈₀ and attRₚₕᵢ₈₀ and a 40 bp sequence homologous to a target chromosome site (Katashkina J I et al. BMC Mol Biol. 2009; 10:34, which is incorporated herein by reference in its entirety), followed by removal according to the previously reported technique of the kanamycin resistant marker (Andreeva I G et al. FEMS Microbiol Lett. 2011; 318(1):55-60, which is incorporated herein by reference in its entirety). SC17(0) is a *λ*Red resistant derivative of P. ananatis AJ13355 (Katashkina J I et al. BMC Mol Biol. 2009; 10:34, which is incorporated herein by reference in its entirety); an annotated complete genome sequence of P. ananatis AJ13355 is available as PRJDA162073 or GenBank Accession Numbers AP012032.1 and AP012033.1. DNA fragments each used for the integration into ampH and ampC genes, respectively were formed by PCR using a plasmid pMWattphi (Minaeva N I et al. BMC Biotechnol. 2008; 8:63, which is incorporated herein by reference in its entirety) as the template and using synthesized oligonucleotides consisting of the nucleotide sequences of SEQ ID NO:23 (primer 13) and SEQ ID NO:24 (primer 14) and synthesized oligonucleotides consisting of the nucleotide sequences of SEQ ID NO:25 (primer 15) and SEQ ID NO:26 (primer 16) as the primers. The resulting chromosomal modification was verified by PCR using synthesized oligonucleotides consisting of the nucleotide sequences of SEQ ID NO:27 (primer 17) and SEQ ID NO:28 (primer 18) and synthesized oligonucleotides consisting of the nucleotide sequences of SEQ ID NO:29 (primer 19) and SEQ ID NO:30 (primer 20) as the primers.

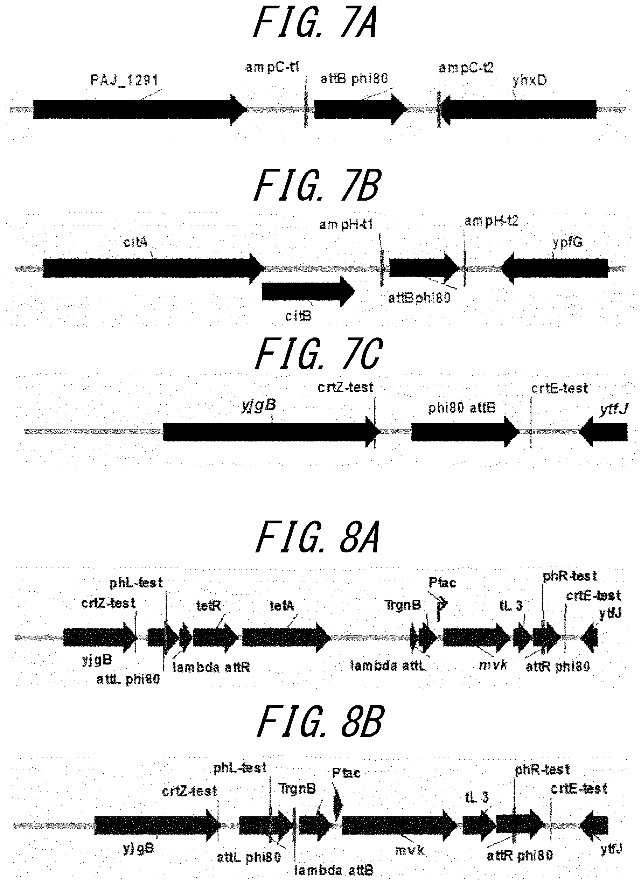

In parallel, a derivative of P. ananatis SC17(0) holding an attB site of the phage phi80 in place of an operon crt (positioned on a megaplasmid pEA320 (320 kb) that is a portion of the genome of P. ananatis AJ13355) was constructed. In order to obtain this strain, the *λ*Red dependent integration of a PCR-amplified DNA fragment holding attLₚₕᵢ₈₀-kan-attRₚₕᵢ₈₀ flanking to a 40 bp region homologous to a target site in the genome was carried out according to the previously described technique (Katashkina J I et al. BMC Mol Biol. 2009; 10:34, which is incorporated herein by reference in its entirety). A DNA fragment used for substitution of the operon crt with attLₚₕᵢ₈₀-kan-attRₚₕᵢ₈₀ was amplified by PCR using synthesized oligonucleotides consisting of the nucleotide sequences of SEQ ID NO:31 (primer 21) and SEQ ID NO:32 (primer 22). The plasmid pMWattphi (Minaeva NI et al. BMC Biotechnol. 2008; 8:63, which is incorporated herein by reference in its entirety) was used as the template in this reaction. The resulting integrant was designated as SC17(0)Δcrt::attLₚₕᵢ₈₀-kan-attRₚₕᵢ₈₀. A chromosomal structure of SC17(0)Δcrt::attLₚₕᵢ₈₀-kan-attRₚₕᵢ₈₀ was verified by PCR using synthesized oligonucleotides consisting of the nucleotide sequences of SEQ ID NO:33 (primer 23) and SEQ ID NO:34 (primer 24). The kanamycin resistant marker was removed from the constructed strain using the helper plasmid pAH129-cat according to the previously reported technique (Andreeva IG et al. FEMS Microbiol Lett. 2011; 318(1):55-60, which is incorporated herein by reference in its entirety). The resulting strain SC17(0)Δcrt::attBₚₕᵢ₈ was verified by PCR using synthesized oligonucleotides consisting of the nucleotide sequences of SEQ ID NO:33 (primer 23) and SEQ ID NO:34 (primer 24). Maps of the resulting modified genomes ΔampC::attBₚₕᵢ₈₀, ΔampH::attBₚₕᵢ₈₀ and Δcrt::attBₚₕᵢ₈₀ are shown in FIGS. 7A, 7B and 7C, respectively.

The above plasmid pAH162-Ptac-mvk (M. paludicola) was integrated into genome of SC17(0)Δcrt::attBₚₕᵢ₈₀ according to the previously reported protocol (Andreeva I G et al. FEMS Microbiol Lett. 2011; 318(1):55-60, which is incorporated herein by reference in its entirety). The integration of the plasmid was confirmed by the polymerase chain reaction using synthesized oligonucleotides consisting of the nucleotide sequences of SEQ ID NO:31 (primer 21), SEQ ID NO:33 (primer 23), SEQ ID NO:32 (primer 22) and SEQ ID NO:34 (primer 24). As a result, a strain SC17(0)Δcrt::pAF[162-P_{tac}-mvk (M. paludicola) was obtained. A map of the modified genome Δcrt::pAH162-P_{tac}-mvk (M. paludicola) is shown in FIG. 8A.

Subsequently, transfer from SC17(0)Δcrt::pAH162-P_{tac}-mvk (M. paludicola) to SC17(0) ΔampC::attBₚₕᵢ₈₀ ΔampH::attBₚₕᵢ₈₀ was carried out via the electroporation of genomic DNA (Katashkina J I et al. BMC Mol Biol. 2009; 10: 34, which is incorporated herein by reference in its entirety). The resulting strain was removed from a vector portion of the integrative plasmid pAH162-P_{tac}-mvk (M. paludicola) using the previously reported helper plasmid pMW-intxis-cat (Katashkina J I et al. BMC Mol Biol. 2009; 10: 34, which is incorporated herein by reference in its entirety). As a result, the marker-deleted strain SC17(0) ΔampH::attB_{*ϕ*80} ΔampC::attB_{*ϕ*80} Δcrt::P_{tac}-mvk (M.paludicola) was obtained. A map of the modified genome Δcrt::P_{tac}-mvk (M.paludicola) is shown in FIG. 8B.

### (1-3-2) Construction of Strain SWITCH-Pₐᵣₐ

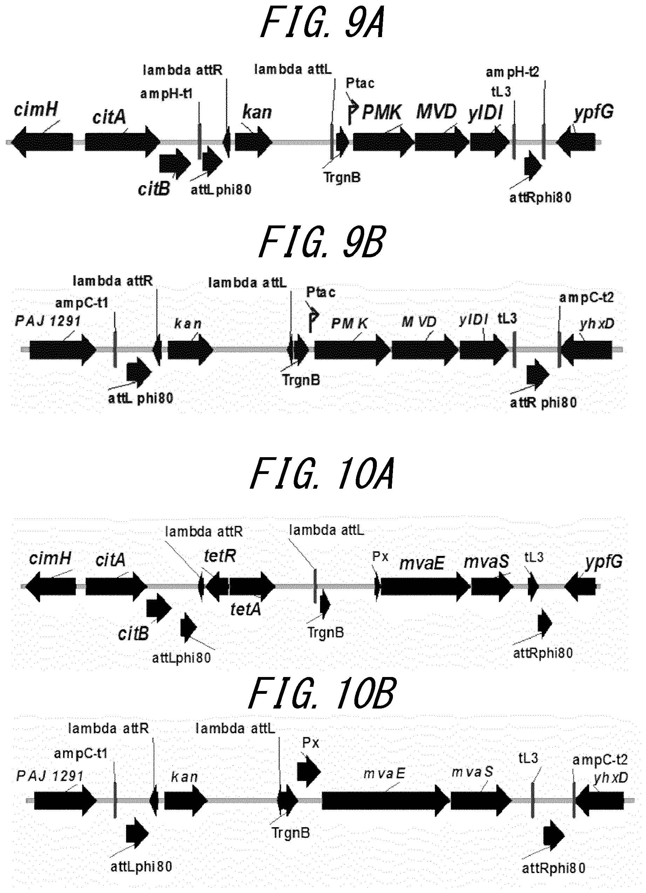

The plasmid pAH162-Km-P_{tac}-KDyI was integrated into a chromosome of the strain SC17(0)ΔampH::attB_{*ϕ*80} ΔampC::attB_{*ϕ*80} Δcrt::P_{tac}-mvk(M.paludicola)/pAH123-cat according to the previously reported protocol (Andreeva I G et al. FEMS Microbiol Lett. 2011; 318(1): 55-60, which is incorporated herein by reference in its entirety). After electrophoresis, cells were seeded on the LB agar containing 50 mg/L of kanamycin. Grown Km^{R} clones were tested by the polymerase chain reactions using synthesized oligonucleotides consisting of the nucleotide sequences of SEQ ID NO:23 (primer 13) and SEQ ID NO:27 (primer 17), and SEQ ID NO:23 (primer 13) and SEQ ID NO:29 (primer 19) as the primers. A strain holding the plasmid pAH162-Km-P_{tac}-KDyI integrated into ΔampH::attB_{*ϕ*80} or pC::attB_{*ϕ*80}m was selected. Maps of the modified genoms ΔampH::pAH162-Km-P_{tac}-KDyI and ΔampC::pAH162-Km-P_{tac}-KDyI are shown in FIGS. 9A and 9B.

pAH162-Pₐᵣₐ-mvaES was inserted into a chromosome of recipient strains SC17(0) ΔampC::pAH162-Km-P_{tac}-KDyI ΔampH::attBₚₕᵢ₈₀ Δcrt::P_{tac}-mvk(M.paludicola) and SC17(0) ΔampC::attBₚₕᵢ₈₀ ΔampH::pAH162-Km-P_{tac}-KDyI Δcrt::P_{tac}-mvk (M.paludicola) using the helper plasmid pAH123-cat according to the previously reported protocol (Andreeva I G et al. FEMS Microbiol Lett. 2011; 318(1): 55-60, which is incorporated herein by reference in its entirety). As a result, two sets of strains designated as SWITCH-Pₐᵣₐ-1 and SWITCH-Pₐᵣₐ-2 were obtained. Maps of the modified genoms ΔampH::pAH162-Pₐᵣₐ-mvaES and ΔampC::pAH162-Pₐᵣₐ-mvaES are shown in FIGS. 10A and 10B.

The plasmid of the isoprene synthase was transduced as follows.

Competent cells of SWITCH-Pₐᵣₐ-1 were adjusted according to an ordinary method, and subsequently pSTV28-P_{tac}-ispSM(US2014113344A1) was introduced thereto, and was evenly applied onto the LB plate containing 60 mg/L of chloramphenicol and cultured 37°C for 18 hours. Subsequently, transformants that exhibited resistance to chloramphenicol were obtained from the resulting plates. The resulting transformants were designated as P.ananatis isoprene-producing bacterium and SWITCH-Pₐᵣₐ-1/pSTV28-P_{tac}-ispSM, respectively.

### (1-4) Production of isoprene via isoprene fermentation

### (1-4-1) Cultivation of P.ananatis isoprene-producing bacterium SWITCH-Pₐᵣₐ-1/pSTV28-P_{tac}-ispSM

SWITCH-Pₐᵣₐ-1/pSTV28-P_{tac}-ispSM was used as a microbe having isoprene productivity. SWITCH-Pₐᵣₐ-1/pSTV28-P_{tac}-ispSM was applied onto an LB plate containing 60 mg/L of chloramphenicol and cultured at 34°C for 16 hours.

0.3 L of the glucose medium as shown in the following Table 1 was placed in three 1 L volume fermenters, respectively, and microbial cells sufficiently grown on one plate were inoculated thereto to start the cultivation. A culture condition was pH 7.0 (controlled with ammonia gas), 30°C, ventilation of 150 mL/minute, and stirring such that an oxygen concentration in the medium was 5% or higher. Note that in the present example, the glucose medium as shown in Table 1 was prepared for 0.15 L of Group A and 0.15 L of Group B, and they were heated and sterilized at 115°C for 10 minutes. After cooling, Group A and Group B were mixed, and chloramphenicol (60 mg/L) was added thereto to use as the medium.

**[Table 1]**

| Group A | |
|---|---|
| Component | Concentration |
| Glucose | 40 g/L |
| Magnesium sulfate heptahydrate | 2.0 g/L |

| Group B | |
|---|---|
| Component | Concentration |
| Ammonium sulfate | 2.0 g/L |
| Potassium dihydrogenphosphate | 2.0 g/L |
| Iron sulfate heptahydrate | 20 mg/L |
| Manganese sulfate pentahydrate | 20 mg/L |
| Yeast extracts | 4.0 g/L |

When all of the glucose contained in the culture medium was consumed, 400 mL of the culture broth was charged into 20 L of a glucose culture medium described in Table 2 below in two fermentation tanks having a volume of 50 L, respectively. A culture condition was pH 7.0 (controlled with ammonia gas), 30°C, ventilation of 10 L/minute, and stirring such that an oxygen concentration in the medium was 5% or higher. Note that in the present example, the glucose medium as shown in Table 2 was prepared for 10 L of Group A and 10 L of Group B, and they were heated and sterilized at 115°C for 10 minutes. After cooling, Group A and Group B were mixed, and chloramphenicol (60 mg/L) was added thereto to use as the medium. During the culture, glucose adjusted to have a concentration of 500 g/L was continuously added so that the glucose concentration of the culture medium was kept at 10 g/L or more.

**[Table 2]**

| Group A | |
|---|---|
| Component | Concentration |
| Glucose | 80 g/L |
| Magnesium sulfate heptahydrate | 2.0 g/L |

| Group B | |
|---|---|
| Component | Concentration |
| Ammonium sulfate | 2.0 g/L |
| Potassium dihydrogenphosphate | 2.0 g/L |
| Iron sulfate heptahydrate | 20 mg/L |
| Manganese sulfate pentahydrate | 20 mg/L |
| Yeast extracts | 4.0 g/L |

The SWITCH-Pₐᵣₐ-1/pSTV28-P_{tac}-ispSM used in this example expresses the mevalonate pathway upstream gene under the arabinose inducible promoter and thus the isoprene production amount was significantly increased by the presence of L-arabinose (manufactured by Wako Pure Chemical Industries, Ltd.). In this Example, L-arabinose was added to the glucose culture medium described in Table 2 so that the final concentration of L-arabinose was 20 mM and whereby the isoprene production phase was induced.

The isoprene concentration of the fermented gas was measured with a multi gas analyzer ("F10", manufactured by GASERA Ltd.). Isoprene was detected after 6 hours from the start of the culture. The isoprene concentration of the fermented gas reached about 800 ppm after 30 hours from the start of the culture. Thereafter, the fermented gas containing isoprene at a concentration of about 800 ppm was supplied to the isoprene recovery apparatus until 76 hours after the start of the culture. At such time, the culture was terminated.

### (1-4-2) Recovery of isoprene contained in fermented gas

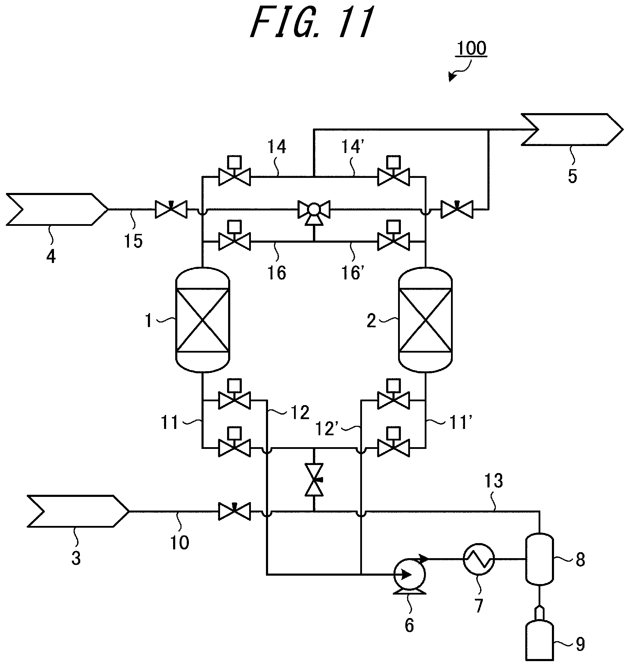

In recovery of isoprene contained in fermented gas, isoprene recovery apparatus illustrated in FIG. 11 was used. A porous adsorbent layers (not illustrated) using a hydrophobic silica gel (S-6, manufactured by Fuji Silysia Chemical Ltd.) was provided in each of a first adsorption tower 1 and a second adsorption tower 2. The porous adsorbent layers were provided in a pre-coated state by blowing with gas having an isoprene concentration of 1.0% by volume.

In the adsorption step, the fermented gas generated from the fermentation tank was used as the feed gas 3. After the feed gas 3 was subjected to dehydration treatment and organic substance removal treatment, the treated feed gas 3 was supplied to the first adsorption tower 1 (or the second adsorption tower 2) through the feed gas supply pipes 10 and 11 (or 10 and 11'). Conditions at the time of adsorption were a temperature of 25°C, a pressure of 101.3 kPa, and a feed gas linear velocity of 1.8 cm/sec. The isoprene concentration of the discharged gas 5 emitted to the atmosphere after the adsorption step was measured with a multi-gas analyzer (F10, manufactured by GASERA).

In a desorption step, nitrogen gas was used as the purge gas 4, and the purge gas 4 was supplied to the first adsorption tower 1 (or the second adsorption tower 2) through purge gas supply pipes 15 and 16 (or 15 and 16'). At the time of desorption, the pressure in the adsorption towers 1, 2 was reduced to 3.3 kPa with the vacuum pump 6. Moreover, the linear velocity of purge gas was 1.6 cm/sec. Cooling water having a temperature of 10°C was circulated in a condenser 7 to cool the gas containing isoprene. Thereafter, the cooled gas was separated with a separator 8 to recover the liquid isoprene 9.

Note that the isoprene recovery apparatus 100 may, as illustrated in FIG. 11, be provided with a return pipe 13 and discharge pipes 14, 14'.

The isoprene recovery apparatus 100 was operated out by alternately switching the adsorption step and the desorption step (the number of iterations of each step: 300 times; switching time of each step: 15 minutes).

Via the operations above, 111g in total of isoprene was recovered. The recovered isoprene (isoprene-containing composition) was used in study of polymerization reaction.

### (2) Purification of isoprene-containing composition

The isoprene prepared with the aforementioned fermentation method was purified with column chromatography. As a column filler (solid), used was an activated alumina (alumina oxide (active) (granular), manufactured by Kanto Chemical Co., Inc.), and molecular sieves (molecular sieves 4A, manufactured by Kanto Chemical Co., Inc.), and as an elute (liquid), used was hexane.

Details of the purification condition were as shown in Table 3 (described below).

### (3) Analysis of isoprene-containing composition

In order to certify the quality of the isoprene provided to the following manufacture of polyisoprene, the isoprene prepared with the aforementioned fermentation method was analyzed with gas chromatography.

The samples were analyzed by using a capillary column for gas chromatography (HP-5MSUI (length: 30 m, internal diameter: 0.25 mm, film thickness: 1µm), manufactured by Agilent Technologies, Inc.) and a gas chromatography apparatus equipped with a hydrogen flame ionization detector (FID) (7820A, manufactured by Agilent Technologies, Inc.) under a condition of: holding at a column temperature of 35°C for 3 minutes, then heating to 300°C at 25°C/min, and then holding for 13 minutes at 300°C; pressure: 19kPa; column flow: 3mL/min; influx method: split 100:1; injection volume of sample into column: 0.1µL; inlet temperature: 250°C;detector temperature: 230°C.

In the present example, in particular, ethyl acetate and 3-methylfuran was analyzed as main components of the impurities. In the case where both ethyl acetate and 3-methylfuran were detected, the total amount thereof was calculated, and in the case where either one was detected, the amount of each was calculated respectively (see Table 3).

A commercially available isoprene (proportion: 0.681) was diluted 10, 100, 1000, 10000 and 100000 times with cooled methanol, and adjusted with a isoprene solution for standard sample. Subsequently, 1 µL of each solution for standard sample was respectively added into vials added with 1 mL of water, to thereby obtain isoprene standard samples.

### (4) Manufacture of polyisoprene

3.5 g of purified isoprene was added into a sufficiently dried 2L stainless reactor.

On the other hand, within a globe box under a nitrogen atmosphere, in a glass container, 40.0 µmol of tris bistrimethylsilylamide gadolinium (Gd[N(SiMe₃)₂]₃) (component (A)), 200 µmol of tributyl aluminum (component (B)), 40.0 µmol of bis(2-diphenylphosphinophenyl) amine (BDPA), 5 g of toluene, 40.0 µmol of triphenylcarbonium tetrakis(pentafluorophenyl) borate (Ph₃C⁺B⁻(C₆F₅)₄) (component (E)) and 35 g of n-hexane were added as the polymerization catalyst composition.

Then, the polymerization catalyst composition was removed from the glove box, and was added into the 2L reactor containing isoprene. The reaction system was maintained at 50°C for 120 minutes to perform polymerization reaction of isoprene. Subsequently, by adding a large amount of methanol into the reactor, the polymerization reaction was terminated, the reaction product was precipitated and separated, and further vacuum-dried at 50°C, to obtain a polymer A (yield: 3.3 g).

By using a gel permeation chromatography (GPC) (GPC apparatus: HLC-8220GPC manufactured by Tosoh Corporation, column: 2 TSKgel GMH_{XL} manufactured by Tosoh Corporation, detector: a differential refractometer (RI)), on the basis of monodisperse polystyrene, the number average molecular weight (Mn) and the molecular weight distribution (Mw/Mn) of polystyrene equivalent of the manufactured polyisoprene were calculated. Note that the measurement temperature was 40°C, and THF was used as an elution solvent.

A rubber composition containing the manufactured polyisoprene was prepared, and by vulcanizing the rubber composition, a vulcanized rubber was obtained. Then, a sample of this vulcanized rubber was subjected to tensile test according to JIS K 6251, and to measure its tensile breaking strength (TB) at room temperature. The results thereof are shown as being indexed with a score of 100 representing Comparative Example 1. The result was as shown in Table 3. An index serving as a comparative evaluation was calculated with a score of 100 representing a conventional example. The evaluation results were as shown in Table 3. A higher index value indicates a higher tensile breaking strength (TB).

**[Table 3]**

| | | | | Comparative Example 1 | Comparative Example 2 | Comparative Example 3 | Comparative Example 4 | Example 1 | Example | Example 3 | Example 4 | Example 5 | Example 6 | Example 7 | Example 8 | Example 9 | Example 10 | Example 11 |
|---|---|---|---|---|---|---|---|---|---|---|---|---|---|---|---|---|---|---|
| | | Type of isoprene | | Synthesized | Synthesized | Fermented | Fermented | Fermented | Fermented | Fermented | Fermented | Fermented | Fermented | Synthesized | Synthesized | Fermented | Fermented | Fermented |
| | | Degree of isoprene purification | | Highly purified | Highly purified | Highly purified | Roughly purified | Roughly purified | Roughly purified | Roughly purified | Roughly purified | Roughly purified | Roughly purified | Highly purified | Highly purified | Roughly purified | Roughly purified | Roughly purified |
| | | | Activated alumina amount (g) | - | - | 15 | 5 | 5 | 15 | 5 | 5 | 5 | 5 | - | - | 15 | 5 | 5 |
| | Isoprene-containing composition | Condition of isoprene purification | Silica gel amount (g) | - | - | 15 | 5 | 5 | 15 | 5 | 5 | 5 | 5 | - | - | 15 | 5 | 5 |
| | | | Purification time (time) | - | - | 1 | 1 | 1 | 0.5 | 0.5 | 0.35 | 1 | 1 | - | - | 0.5 | 0.5 | 1 |
| | | | Purification temperature (°C) | - | - | RT | RT | RT | RT | RT | RT | RT | RT | - | - | RT | RT | RT |
| | | Isoprene | Isoprene (mass%) | 100 | 100 | 99.97 | 99.75 | 99.75 | 99.95 | 99.0 | 98.4 | 99.75 | 99.75 | 99.75 | 99.75 | 99.95 | 99.0 | 99.75 |
| | | | Ethyl acetate and 3-methylfum (total) (mass%) | 0 | 0 | 0.03 | 0.25 | 0.25 | 0.05 | 1.0 | 1.6 | 0.25 | 0.25 | - | - | 0.05 | 1.0 | 0.25 |
| | | Condition of isoprene purification | Ethylacetate (mass%) | 0 | 0 | - | - | - | - | - | - | - | - | 0.25 | 0 | - | - | - |
| | | | 3-methylfuran (mass%) | 0 | 0 | - | - | - | - | - | - | - | - | 0 | 0.25 | - | - | - |
| | | | Total (mass%) | 100 | 100 | 100 | 100 | 100 | 100 | 100 | 100 | 100 | 100 | 100 | 100 | 100 | 100 | 100 |
| Specs of method for producing poly isoprene | | Rare earth element compound Component (A) | Gd[N(SiMe₃)₂]₃ (µmol) | 40 | 40 | 40 | 40 | 40 | 40 | 40 | 40 | 40 | 40 | 40 | 40 | 40 | 40 | 0 |
| | | | Versatic acid Nd (µmol) | 0 | 0 | 0 | 0 | 0 | 0 | 0 | 0 | 0 | 0 | 0 | 0 | 0 | 0 | 40 |
| | | | TrⁱBuAl (µmol) | 100 | 200 | 100 | 100 | 200 | 200 | 200 | 300 | 150 | 300 | 200 | 200 | 150 | 300 | 200 |
| | | Organometallic compound Component (B) | TrⁱBuAl (g) | 002 | 0.04 | 0.02 | 0.02 | 0.04 | 0.04 | 0.04 | 0.06 | 0.03 | 0.06 | 0.04 | 0.04 | 0.03 | 0.06 | 0.04 |
| | | | Isoprene-containing composition (g) | 3.5 | 3.5 | 3.5 | 3.5 | 3.5 | 3.5 | 3.5 | 3.5 | 3.5 | 3.5 | 3.5 | 3.5 | 3.5 | 3.5 | 3.5 |
| | | | Ratio of TriBuAl to isoprene-containing composition (parts by mass) | 0.57 | 1.13 | 0.57 | 0.57 | 1.13 | 1.13 | 1.13 | 1.70 | 0.85 | 1.70 | 1.13 | 1.13 | 0.85 | 1.70 | 1.13 |
| | Polymerization caalyst composition | Ionic compowd Component (E) | Ph₃CB(C₆F₅)₄ (µmol) | 40 | 40 | 40 | 40 | 40 | 40 | 40 | 40 | 40 | 40 | 40 | 40 | 40 | 40 | 40 |
| | | Compound capable of serving as an anionic ligand Component (F) | BDPA (µmol) | 40 | 40 | 40 | 40 | 40 | 40 | 40 | 40 | 40 | 40 | 40 | 40 | 40 | 40 | 40 |
| | | Solvent | Toluene (g) | 5 | 5 | 5 | 5 | 5 | 5 | 5 | 5 | 5 | 5 | 5 | 5 | 5 | 5 | 5 |
| | | | Hexane (g) | 35 | 35 | 35 | 35 | 35 | 35 | 35 | 35 | 35 | 35 | 35 | 35 | 35 | 35 | 35 |
| | | | Capability of polymerization | Capable | Capable | Capable | Incapable | Capable | Capable | Capable | Capable | Capable | Capable | Capable | Capable | Capable | Capable | Capable |
| Specs of polyisoprene. | | | Mn (×10³) | 1220 | 1150 | 1190 | - | 1640 | 1250 | 1300 | 1250 | 1320 | 1360 | 1620 | 1590 | 1553 | 1515 | 1580 |
| | | | Mw (×10³) | 2367 | 2220 | 2309 | - | 3150 | 2450 | 2522 | 2058 | 2530 | 2665 | 3159 | 3159 | 3028 | 3015 | 3239 |
| | | | Mw/Mn | 1.94 | 1.93 | 1.94 | - | 1.92 | 1.96 | 1.94 | 1.65 | 1.92 | 1.96 | 1.95 | 1.99 | 1.95 | 1.99 | 2.05 |
| | | | TB | 100 | 99 | 100 | - | 110 | 100 | 102 | 100 | 105 | 105 | 107 | 107 | 107 | 106 | 110 |

### (Examples 2 to 11, Comparative Examples 1 to 4)

The manufacture of polyisoprene was performed the same as Example 1 except for the points of condition as shown in Table 3.

Comparison of Examples 1 to 11 and Comparative Examples 1 to 4 showed that if the compounding amount of the organometallic compound was 0.6 to 3.0 parts by mass per 100 parts by mass of the isoprene-containing composition, a polyisoprene having a comparatively high molecular weight may be manufactured, and thereby, it was possible to obtain the desired effect of this disclosure, such that the strength of the manufactured synthesized polyisoprene may be enhanced. Here, the comparison between Examples 1 to 3, 5 to 11 and Example 4 showed that if the compounding amount of the oxygen-containing neutral compound was 0.05 to 1.0 parts by mass per 100 parts by mass of the isoprene-containing composition, the aforementioned effect of this disclosure was particularly significant.

Moreover, the comparison of the comparison result of Examples 1 to 6, 9 to 11 and Comparative Example 4 and the comparison result of Examples 7, 8 and Comparative Examples 1, 2 showed that the aforementioned effect of this disclosure may be obtained beneficially particularly in the case of using a fermented isoprene in which an oxygen-containing neutral compound remains after the purification.

### INDUSTRIAL APPLICABILITY

According to the method for producing polyisoprene of this disclosure, it is possible to produce a polyisoprene having a comparatively high molecular weight, and to thereby improve the produced synthesized polyisoprene.

### REFERENCE SIGNS LIST

- 1: first adsorption tower
- 2: second adsorption tower
- 3: fermented gas containing isoprene (feed gas)
- 4: purge gas
- 5: discharged gas
- 6: vacuum pump
- 7: condenser
- 8: separator
- 9: recovered isoprene (liquid isoprene)
- 10,11,11': feed gas supply pipe
- 12,12': isoprene-containing purge gas supply pipe
- 13: return pipe
- 14,14': discharge pipe
- 15,16,16': purge gas supply pipe
- 100: isoprene recovery apparatus

### [Indication of microbe deposit]

Biologicals depository: VKPM Russian National Collection of Industrial Microorganisms
Addressing: FGUP GosNII Genetika, 1 Dorozhny proezd, 1, Moscow 117545, Russian Federation
Deposit number: VKPM B-9246
Deposit date: September 21, 2005

## Claims

1. A method for producing polyisoprene comprising: polymerizing isoprene by using an isoprene-containing composition containing isoprene and an oxygen-containing neutral compound, and a polymerization catalyst composition containing a rare earth element compound and an organometallic compound, wherein:
a compounding amount of the organometallic compound is 0.6 to 3.0 parts by mass per 100 parts by mass of the isoprene-containing composition.

2. The method for producing polyisoprene according to claim 1, wherein:
a compounding amount of the oxygen-containing neutral compound is 0.05 to 1.0 parts by mass per 100 parts by mass of the isoprene-containing composition.

3. The method for producing polyisoprene according to claim 1 or 2, wherein:
the isoprene-containing composition is prepared via a fermentation method.

4. The method for producing polyisoprene according to any one of claims 1 to 3, wherein:
the oxygen-containing neutral compound is selected from the group consisting of an ether, an ester or a ketone.

5. The method for producing polyisoprene according to any one of claims 1 to 4, wherein:
a number-average molecular weight Mn of the polyisoprene is 1,000,000 or more.

6. The method for producing polyisoprene according to any one of claims 1 to 5, wherein:
the number-average molecular weight Mn of the polyisoprene is 1,300,000 or more.

7. The method for producing polyisoprene according to any one of claims 1 to 6, wherein:
a molecular weight distribution Mw/Mn of the polyisoprene is 2.5 or less.

8. The method for producing polyisoprene according to any one of claims 1 to 7, wherein:
a cis-1,4-bond content of the polyisoprene is 98% or more.

9. The method for producing polyisoprene according to any one of claims 1 to 8, wherein:
the rare earth element compound is a rare earth element-containing compound or a reaction product of the rare earth element-containing compound and a Lewis base, the rare earth element-containing compound or reaction product having metal-nitrogen bond (M-N bond), and
the rare earth element-containing compound is a compound containing scandium, yttrium or a lanthanoid element selected from elements of atomic numbers 57 to 71.

10. The method for producing polyisoprene according to any one of claims 1 to 9, wherein:
the organometallic compound is a compound represented with the following formula (S2)
YR⁴ₐR⁵_{b}R⁶_{c}... (S2)
where Y is a metallic element selected from the group consisting of Group 1, Group 2, Group 12 and Group 13; R⁴ and R⁵ are C1 to C10 hydrocarbon group or hydrogen atom, and may be either identical or different; R⁶ is a C1 to C10 hydrocarbon group; R⁶ may be either identical to or different from the R⁴ or R⁵; if Y is a Group 1 metallic element, a is 1, and b, c are 0; if Y is a Group 2 metallic element or a Group 12 metallic element, a and b are 1, and c is 0; and if Y is a Group 13 metallic element, a, b and c are 1; or
the following formula (S3)
AlR⁷R⁸R⁹ ... (S3)
where R⁷ and R⁸ are C1 to C10 hydrocarbon group or hydrogen atom, and may be either identical or different; R⁹ is a C1 to C10 hydrocarbon group; and R⁹ may be either identical to or different from the R⁷ or R⁸.

11. The method for producing polyisoprene according to any one of claims 1 to 10, wherein:
the polymerization catalyst composition is at least one compound selected from the group consisting of an aluminoxane compound, a halogen compound, an ionic compound, or a compound capable of serving as an anionic ligand.

## Patentansprüche

1. Verfahren zur Herstellung von Polyisopren, umfassend: Polymerisieren von Isopren durch Verwenden einer Isopren enthaltenden Zusammensetzung, die Isopren und eine sauerstoffhaltige neutrale Verbindung enthält, und einer Polymerisationskatalysatorzusammensetzung, die eine Seltenerdeelementverbindung und eine organometallische Verbindung enthält, wobei:
eine Compoundiermenge der organometallischen Verbindung 0,6 bis 3,0 Masseteile pro 100 Masseteile der Isopren enthaltenden Zusammensetzung beträgt.

2. Verfahren zur Herstellung von Polyisopren nach Anspruch 1, wobei:
eine Compoundiermenge der sauerstoffhaltigen neutralen Verbindung 0,05 bis 1,0 Masseteile pro 100 Masseteile der Isopren enthaltenden Zusammensetzung beträgt.

3. Verfahren zur Herstellung von Polyisopren nach Anspruch 1 oder 2, wobei:
die Isopren enthaltende Zusammensetzung durch ein Fermentationsverfahren hergestellt wird.

4. Verfahren zur Herstellung von Polyisopren nach irgendeinem der Ansprüche 1 bis 3, wobei:
die sauerstoffhaltige neutrale Verbindung aus der Gruppe ausgewählt wird bestehend aus einem Ether, Ester oder einem Keton.

5. Verfahren zur Herstellung von Polyisopren nach irgendeinem der Ansprüche 1 bis 4, wobei:
ein zahlendurchschnittliches Molekulargewicht Mn des Polyisoprens 1.000.000 oder mehr beträgt.

6. Verfahren zur Herstellung von Polyisopren nach irgendeinem der Ansprüche 1 bis 5, wobei:
das zahlendurchschnittliche Molekulargewicht Mn des Polyisoprens 1.300.000 oder mehr beträgt.

7. Verfahren zur Herstellung von Polyisopren nach irgendeinem der Ansprüche 1 bis 6, wobei:
eine Molekulargewichtsverteilung Mw/Mn des Polyisoprens 2,5 oder weniger beträgt.

8. Verfahren zur Herstellung von Polyisopren nach irgendeinem der Ansprüche 1 bis 7, wobei:
ein cis-1,4-Bindungsgehalt des Polyisoprens 98 % oder mehr beträgt.

9. Verfahren zur Herstellung von Polyisopren nach irgendeinem der Ansprüche 1 bis 8, wobei:
die Seltenerdeelementverbindung eine Seltenerdeelement enthaltende Verbindung oder ein Reaktionsprodukt der Seltenerdeelement enthaltende Verbindung und einer Lewis-Base ist, wobei die Seltenerdeelement enthaltende Verbindung oder das Reaktionsprodukt eine Metall-Stickstoffbindung (M-N-Bindung) aufweist und
die Seltenerdeelement enthaltende Verbindung eine Verbindung ist, die Scandium, Yttrium oder ein Lanthanoidelement ausgewählt unter Elementen der Atomzahlen 57 bis 71 enthält.

10. Verfahren zur Herstellung von Polyisopren nach irgendeinem der Ansprüche 1 bis 9, wobei:
die organometallische Verbindung eine Verbindung ist, die durch die folgende Formel (S2) dargestellt ist
YR⁴ₐR⁵_{b}R⁶_{c}...... (S2)
wobei Y ein metallisches Element ist ausgewählt aus der Gruppe bestehend aus Gruppe 1, Gruppe 2, Gruppe 12 und Gruppe 13; R⁴ und R⁵ eine C1- bis C10-Kohlenwasserstoffgruppe oder ein Wasserstoffatom sind und entweder identisch oder verschieden sein können; R⁶ eine C1-bis C10-Kohlenwasserstoffgruppe ist; R⁶ entweder identisch mit oder verschieden von R⁴ oder R⁵ sein kann; wenn Y ein metallisches Element der Gruppe 1 ist; beträgt a 1 und b, c betragen 0; wenn Y ein metallisches Element der Gruppe 2 oder ein metallisches Element der Gruppe 12 ist; betragen a und b 1 und c beträgt 0; und wenn Y ein metallisches Element der Gruppe 13 ist, betragen a, b und c 1; oder
die folgende Formel (S3)
AlR⁷R⁸R⁹... (S3)
wobei R⁷ und R⁸ eine C1- bis C10-Kohlenwasserstoffgruppe oder ein Wasserstoffatom sind und entweder identisch oder verschieden sein können; R⁹ eine C1-bis C10- Kohlenwasserstoffgruppe ist; und R⁹ entweder identisch mit oder verschieden von den R⁷ oder R⁸ sein können.

11. Verfahren zur Herstellung von Polyisopren nach irgendeinem der Ansprüche 1 bis 10, wobei:
die Polymerisationskatalysatorzusammensetzung mindestens eine Verbindung ist ausgewählt aus der Gruppe bestehend aus einer Aluminoxanverbindung, einer Halogenverbindung, einer ionischen Verbindung oder einer Verbindung, die in der Lage ist, als ein anionischer Ligand zu dienen.

## Revendications

1. Procédé de production de polyisoprène comprenant: la polymérisation d'isoprène en utilisant une composition contenant de l'isoprène qui contient de l'isoprène et un composé neutre contenant de l'oxygène, et une composition de catalyseur de polymérisation contenant un composé élément de terres rares et un composé organométallique, où:
une quantité de malaxage du composé organométallique est de 0,6 à 3,0 parties en masse pour 100 parties en masse de la composition contenant de l'isoprène.

2. Procédé de production de polyisoprène selon la revendication 1, où:
une quantité de malaxage du composé neutre contenant de l'oxygène est de 0,05 à 1,0 partie en masse pour 100 parties en masse de la composition contenant de l'isoprène.

3. Procédé de production de polyisoprène selon la revendication 1 ou 2, où:
la composition contenant de l'isoprène est préparée par l'intermédiaire d'un procédé de fermentation.

4. Procédé de production de polyisoprène selon l'une quelconque des revendications 1 à 3, où:
le composé neutre contenant de l'oxygène est sélectionné dans le groupe constitué d'un éther, d'un ester ou d'une cétone.

5. Procédé de production de polyisoprène selon l'une quelconque des revendications 1 à 4, où:
un poids moléculaire moyen en nombre Mn du polyisoprène est de 1 000 000 ou plus.

6. Procédé de production de polyisoprène selon l'une quelconque des revendications 1 à 5, où:
le poids moléculaire moyen en nombre Mn du polyisoprène est de 1 300 000 ou plus.

7. Procédé de production de polyisoprène selon l'une quelconque des revendications 1 à 6, où:
une distribution des poids moléculaires Mw/Mn du polyisoprène est de 2,5 ou moins.

8. Procédé de production de polyisoprène selon l'une quelconque des revendications 1 à 7, où:
une teneur en liaisons cis-1,4 du polyisoprène est de 98 % ou plus.

9. Procédé de production de polyisoprène selon l'une quelconque des revendications 1 à 8, où:
le composé élément de terres rares est un composé contenant un élément de terres rares ou un produit réactionnel du composé contenant un élément de terres rares et une base de Lewis, le composé contenant un élément de terres rares ou le produit réactionnel ayant une liaison métal-azote (liaison M-N), et
le composé contenant un élément de terres rares est un composé contenant du scandium, de l'yttrium ou un élément lanthanoïde sélectionné parmi les éléments des numéros atomiques 57 à 71.

10. Procédé de production de polyisoprène selon l'une quelconque des revendications 1 à 9, où:
le composé organométallique est un composé représenté avec la formule suivante (S2)
YR⁴ₐR⁵_{b}R⁶_{c} ... (S2)
où Y est un élément métallique sélectionné dans le groupe constitué du Groupe 1, Groupe 2, Groupe 12 et Groupe 13; R⁴ et R⁵ sont un groupe hydrocarbure en C1 à C10 ou un atome d'hydrogène, et peuvent être soit identiques soit différents; R⁶ est un groupe hydrocarbure en C1 à C10; R⁶ peut être soit identique soit différent de R⁴ ou R⁵; si Y est un élément métallique du Groupe 1, a présente la valeur de 1, et b, c ont la valeur de 0; si Y est un élément métallique du Groupe 2 ou un élément métallique du Groupe 12, a et b ont la valeur de 1, et c a la valeur de 0; et si Y est un élément métallique du Groupe 13, a, b et c ont la valeur de 1; ou
la formule suivante (S3)
AlR⁷R⁸R⁹ ... (S3)
où R⁷ et R⁸ sont un groupe hydrocarbure en C1 à C10 ou un atome d'hydrogène, et peuvent être soit identiques soit différents; R⁹ est un groupe hydrocarbure en C1 à C10; et R⁹ peut être soit identique soit différent de R⁷ ou R⁸.

11. Procédé de production de polyisoprène selon l'une quelconque des revendications 1 à 10, où:
la composition de catalyseur de polymérisation est au moins un composé sélectionné dans le groupe constitué d'un composé aluminoxane, d'un composé halogène, d'un composé ionique, ou d'un composé capable de servir de ligand anionique.
